(19) Europäisches Patentamt
European Patent Office
Office européen des brevets

(11) EP 4 495 104 A1

(12) **EUROPEAN PATENT APPLICATION**
published in accordance with Art. 153(4) EPC

(43) Date of publication:
**22.01.2025 Bulletin 2025/04**

(21) Application number: **23770898.7**

(22) Date of filing: **16.03.2023**

(51) International Patent Classification (IPC):
***C07D 233/58*** *(2006.01)* ***C08B 3/06*** *(2006.01)*
***C08B 3/22*** *(2006.01)*

(52) Cooperative Patent Classification (CPC):
**C07D 233/58; C08B 3/06; C08B 3/22**

(86) International application number:
**PCT/JP2023/010486**

(87) International publication number:
**WO 2023/176945 (21.09.2023 Gazette 2023/38)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.03.2022 PCT/JP2022/011991**

(71) Applicants:
- **National University Corporation Kanazawa University**
  **Ishikawa 920-1192 (JP)**
- **Daicel Corporation**
  **Osaka-shi, Osaka 530-0011 (JP)**

(72) Inventors:
- **WADA, Naoki**
  **Kanazawa-shi, Ishikawa 920-1192 (JP)**
- **TAKAHASHI, Kenji**
  **Kanazawa-shi, Ishikawa 920-1192 (JP)**
- **OKUBO, Yuto**
  **Tokyo 108-8230 (JP)**
- **WATANABE, Susumu**
  **Tokyo 108-8230 (JP)**
- **WATANABE, Hitoshi**
  **Tokyo 108-8230 (JP)**
- **ARAI, Takashi**
  **Tokyo 108-8230 (JP)**

(74) Representative: **Grünecker Patent- und Rechtsanwälte**
**PartG mbB**
**Leopoldstraße 4**
**80802 München (DE)**

# (54) METHOD FOR RECOVERING IONIC LIQUID

(57) Provided is a method for easily and efficiently recovering an ionic liquid from a liquid mixture containing the ionic liquid and an organic acid. The method for recovering an ionic liquid is characterized by subjecting a liquid mixture (A) containing an ionic liquid, an organic acid, and a solvent (provided that an organic acid is excluded) to distillation.

EP 4 495 104 A1

## Description

### Technical Field

The present disclosure relates to a method for recovering an ionic liquid by distillation.

### Background Art

In recent years, ionic liquids have been proposed as solvents for dissolving a biomass containing a polysaccharide such as cellulose or lignocellulose (hereinafter may be referred to as "polysaccharide-containing biomass"), and there have been introduced techniques for derivatizing a polysaccharide under a homogeneous reaction system by using an ionic liquid. Because of its very low volatility, an ionic liquid does not pose a risk associated with volatilization, such as contamination or inflammation. In addition, an ionic liquid has a strong ability to dissolve cellulose or the like, and thus it is under research and development as a solvent used for processing a polysaccharide.

For example, Example 1 of Patent Document 1 describes that 120 mg of bagasse (sugar cane residue) was dissolved in 4 g of 1-ethyl-3-methylimidazolium acetate, which is an ionic liquid (bagasse concentration in the ionic liquid: 3 wt.%), the solution was vacuum-dried at 80°C and under stirring conditions overnight, and then 4 mL of isopropenyl acetate was added to the reaction system, the resulting reaction solution was reprecipitated in methanol, and a solid polysaccharide derivative (cellulose acetate) was obtained by filtration.

Since an ionic liquid is very expensive, there has been studied a method for recovering and reusing the ionic liquid after completion of reaction. For example, Patent Document 2 describes a method for separating and recovering an ionic liquid from a mixed solution containing the ionic liquid and two or more dissolved solutes by using an ion-exchange membrane. Patent Document 3 describes a method for separating and recovering an ionic liquid by crystallization and subsequent washing with a solvent such as ethyl acetate, the ionic liquid containing, as a cationic component, a quaternary ammonium skeleton having an alkoxyalkyl group or a nitrogen-containing five-membered heterocyclic skeleton having an alkoxyalkyl group and containing an amino group as an anionic component. Patent Document 4 describes a method in which an ionic liquid containing impurities is dissolved in a mixed solvent of a polar aprotic organic solvent and a non-polar organic solvent, and then the temperature of the solution is lowered to allow the ionic liquid to crystallize from the solution, thereby separating and recovering the ionic liquid. Patent Document 5 describes an ionic liquid purification method in which an ionic liquid is partially crystallized from a melt thereof, and a crystalline product is separated from the remaining melt.

### Citation List

#### Patent Document

Patent Document 1: WO 2016/068053
Patent Document 2: JP 2015-96255 A
Patent Document 3: JP 2012-144441 A
Patent Document 4: JP 2010-184902 A
Patent Document 5: JA 2008-523005 A

### Summary of Invention

#### Technical Problem

When cellulose acetate is produced from a polysaccharide-containing biomass as described in Patent Document 1, the polysaccharide-containing biomass is subjected to an esterification reaction in the presence of the polysaccharide-containing biomass as a raw material, an esterifying agent, and an ionic liquid as a solvent. However, an organic acid derived from the esterifying agent is produced as a by-product of the reaction. Therefore, the reaction solution after the esterification reaction is a mixture containing the organic acid and the ionic liquid. Although the exact reason is not clear, the organic acid is difficult to remove from such a mixture by distillation presumably due to the interaction between the organic acid and the ionic liquid. Thus, the ionic liquid is difficult to separate and recover from the liquid mixture.

Therefore, an object of the present disclosure is to provide a method for easily and efficiently recovering an ionic liquid from a liquid mixture containing the ionic liquid and an organic acid.

Solution to Problem

As a result of extensive studies for solving the above-described problems, the inventors of the present disclosure have found that when a solvent is blended into a liquid mixture containing an ionic liquid and an organic acid, and then the resultant mixture is subjected to distillation, the ionic liquid can be easily and efficiently recovered from the liquid mixture. The invention according to the present disclosure has been accomplished on the basis of these findings.
Accordingly, the present disclosure provides a method for recovering an ionic liquid, the method being characterized by including subjecting a liquid mixture (A) containing an ionic liquid, an organic acid, and a solvent (provided that an organic acid is excluded) to distillation.
In the method according to an embodiment of the present disclosure, preferably, a solvent (provided that an organic acid is excluded) is added to a liquid mixture (B) containing an ionic liquid and an organic acid, and the liquid mixture is subjected to distillation.
The solvent is preferably a polar solvent.
The HSP value [$(MPa)^{0.5}$] of the solvent at 25°C is preferably 15 or more.
The ionic liquid preferably contains an imidazolium cation or a quaternary ammonium cation as a cationic component.
The ionic liquid preferably contains a carboxylate anion as an anionic component.
The organic acid is preferably a carboxylic acid.
The solvent is at least one selected from the group consisting of cyclohexanol, 1-hexanol, 1-heptanol, 2-octanol, and 2-ethylhexanol, and the content of the ionic liquid in the liquid mixture (A) is preferably from 0.3 to 9 wt.%.
The pressure during the distillation is preferably 1519 hPa or less.

Advantageous Effects of Invention

According to the method for recovering an ionic liquid of the present disclosure, an ionic liquid can be easily and efficiently recovered from a liquid mixture containing the ionic liquid and an organic acid.

Description of Embodiments

The method for recovering an ionic liquid of the present disclosure (hereinafter may be referred to as "the method of the present disclosure") is a method for recovering an ionic liquid by subjecting a liquid mixture (A) containing an ionic liquid, an organic acid, and a solvent (provided that an organic acid is excluded) to distillation. The solvent (solvent excluding an organic acid) may be referred to as a solvent (X).

[Liquid mixture]

Hereinafter, a liquid mixture (A) and a liquid mixture (B) will be described. In the present specification, a liquid mixture containing an ionic liquid and an organic acid is referred to as a liquid mixture (B). In the present specification, a liquid mixture containing an ionic liquid, an organic acid, and a solvent (X) is referred to as a liquid mixture (A). The liquid mixture (A) is not particularly limited as long as it contains an ionic liquid, an organic acid, and a solvent (X), but it is preferably a mixture in which the solvent (X) is blended into the liquid mixture (B) containing an ionic liquid and an organic acid. The liquid mixture (B) is not particularly limited as long as it contains an ionic liquid and an organic acid, and may be, for example, a liquid mixture (hereinafter may be referred to as a "liquid mixture (B1)") containing a reaction product obtained upon esterification of a specific raw material with an esterifying agent or the like by using an ionic liquid as a solvent. The liquid mixture (B 1) contains, for example, an esterified product as a target product (esterified product of the raw material described above), an organic acid derived from an esterifying agent, and an ionic liquid. The liquid mixture (B 1) may further contain an unreacted raw material, an unreacted esterifying agent, a by-product other than the organic acid, and a solvent other than the ionic liquid. The liquid mixture (B 1) may be a product prepared by removing some or all of components other than the ionic liquid and the organic acid by a known or customary method. That is, the liquid mixture (B1) only needs to contain at least an organic acid derived from an esterifying agent and an ionic liquid.
Hereinafter, the liquid mixture (B1) will be described with reference to a case where the raw material is a polysaccharide. Specifically, the case refers to an embodiment in which the liquid mixture (B) is a liquid mixture (hereinafter may be referred to as a "liquid mixture (B2)") obtained upon esterification of a polysaccharide with an esterifying agent by using an ionic liquid as a solvent. The liquid mixture (B2) contains, for example, an esterified product of a polysaccharide, i.e., a target product, an organic acid derived from an esterifying agent, and an ionic liquid. The liquid mixture (B2) may further contain an unreacted polysaccharide, an unreacted esterifying agent, a by-product other than the organic acid, and a solvent other than the ionic liquid. The liquid mixture (B2) may be a product prepared by removing some or all of components other than the ionic liquid and the organic acid by a known or customary method.

To summarize the above,

the liquid mixture (B1) is an example of a specific embodiment of the liquid mixture (B), and is a liquid mixture (B) containing a reaction product obtained upon esterification of a specific raw material with an esterifying agent or the like by using an ionic liquid as a solvent, and
the liquid mixture (B2) is an example of a further specific embodiment of the liquid mixture (B1), and is a liquid mixture (B) when a raw material to be esterified with an esterifying agent is a polysaccharide, and
the liquid mixture (B) is a conceptual liquid mixture including the liquid mixture (B1) and the liquid mixture (B2). It is noted again that the liquid mixture (B) is not limited to the liquid mixture (B1) or the liquid mixture (B2) in the present embodiment.

Next will be described in detail the raw material, the esterifying agent, the ionic liquid, and the solvent other than the ionic liquid in the case where the liquid mixture (B) is the liquid mixture (B1).

<Raw material>

A raw material will now be described. The raw material means a starting material to be esterified with an esterifying agent. The raw material is preferably a polysaccharide. As described above, when the raw material is a polysaccharide, the liquid mixture (B1) refers to the liquid mixture (B2).
The polysaccharide may be a biomass containing a polysaccharide (polysaccharide-containing biomass). The polysaccharide-containing biomass is not particularly limited as long as it contains a polysaccharide. Examples of the biomass include bagasse (sugar cane residue); kenaf; woods, such as Japanese cedar, eucalyptus, Japanese red pine, poplar, lauan, Japanese cypress, monarch birch, and Sitka spruce; shells of crustaceans, such as crabs and shrimps; cereals, such as rice, wheat, corn, and sorghum; potatoes and similar plants, such as potato, sweet potato, and cassava; and other cellulose-based plant-derived raw materials (pulp waste liquid, rice straw, rice hull, fruit fiber, fruit kernel shells, such as those of ginkgo nuts, and empty fruit bunches). Pulp obtained by purifying such a biomass can also be used. The polysaccharide-containing biomass may be in a polysaccharide state after being subjected to various pretreatments such as cutting and drying as necessary, and then a process of separation and extraction of a polysaccharide (e.g., cellulose). One type of the polysaccharides described above may be used alone, or two or more types thereof may be used.
The polysaccharide is not particularly limited, and examples thereof include cellulose, hemicellulose, xylan, mannan, glucomannan, glucuronoxylan, starch, amylose, amylopectin, glycogen, dextrin, pectin, chitin, chitosan, agarose, carrageenan, isolichenan, laminaran, lichenan, glucan, inulin, levan, fructan, galactan, arabinan, pentosan, alginic acid, pectic acid, protuberic acid, colominic acid, porphyran, fucoidan, ascophyllan, locust bean gum, guar gum, tamarind gum, tara gum, and gum arabic.

<Esterifying agent>

Next, an esterifying agent will be described. The esterifying agent is a compound for esterifying a raw material such as a polysaccharide.
The esterifying agent is not particularly limited, but a compound corresponding to the type of a target esterified product can be appropriately selected and used. In particular, the esterifying agent is preferably one or more selected from the group consisting of a chain ester compound, a cyclic ester compound, an unsaturated aldehyde, a saturated aldehyde, an acid halide, an acid anhydride, and allyl alcohol. Among these, an acid anhydride is preferred from the viewpoints of handleability and the yield of an esterified product to be produced. One type of the esterifying agents may be used, or two or more types thereof may be used in combination.
The chain ester compound is not particularly limited, and examples thereof include compounds, for example, carboxylic acid alkyl esters such as methyl acetate, and carboxylic acid alkenyl esters such as isopropenyl acetate and vinyl acetate.
The cyclic ester compound is not particularly limited, and examples thereof include lactones such as β-propiolactone, δ-valerolactone, γ-butyrolactone, ε-caprolactone, α,α-dimethyl-β-propiolactone, β-ethyl-δ-valerolactone, α-methyl-ε-caprolactone, β-methyl-ε-caprolactone, γ-methyl-ε-caprolactone, and 3,3,5-trimethyl-ε-caprolactone; and lactides such as glycolide and lactide.
The unsaturated aldehyde is not particularly limited, and examples thereof include 2-butenal, 2-hexenal, 2-decenal, 2-undecenal, 2-dienal, 2,4-heptadienal, 2,4-decadienal, and aromatic aldehydes such as cinnamaldehyde and benzaldehyde.
The saturated aldehyde is not particularly limited, and examples thereof include propanal, hexanal, octanal, and nonanal.
The acid halide is not particularly limited, and examples thereof include carboxylic halides such as carboxylic

fluoride, carboxylic chloride, carboxylic bromide, and carboxylic iodide. Specific examples of the carboxylic halide include acetyl fluoride, acetyl chloride, acetyl bromide, acetyl iodide, propionyl fluoride, propionyl chloride, propionyl bromide, propionyl iodide, butyryl fluoride, butyryl chloride, butyryl bromide, butyryl iodide, benzoyl fluoride, benzoyl chloride, benzoyl bromide, and benzoyl iodide.

The acid anhydride is not particularly limited, and examples thereof include acetic anhydride, propionic anhydride, butyric anhydride, valeric anhydride, caproic anhydride, enanthic anhydride, caprylic anhydride, pelargonic anhydride, capric anhydride, lauric anhydride, myristic anhydride, palmitic anhydride, stearic anhydride, oleic anhydride, linoleic anhydride, linolenic anhydride, benzoic anhydride, phthalic anhydride, maleic anhydride, and succinic anhydride. Among these, acetic anhydride is particularly preferred.

The allyl alcohol is not particularly limited, and examples thereof include methallyl alcohol, acrylic alcohol, 2-hydroxymethyl-1-butene, and $\alpha$-hydroxymethylstyrene.

<Ionic liquid>

Next, an ionic liquid will be described. The ionic liquid is a solvent for dissolving and/or dispersing a raw material (e.g., polysaccharide) (in particular, a solvent for dissolving a raw material). Specific examples of the ionic liquid will be described below in the section [Ionic liquid]. Thus, specific exemplification of the ionic liquid is omitted here. The ionic liquid to be used may be any of the specific ionic liquids exemplified in the section [Ionic liquid].

<Solvent other than ionic liquid [Solvent (S)]>

Next, a solvent other than the ionic liquid will be described. In the present specification, the above-described solvent may be referred to as a "solvent (S)". The solvent (S) is a solvent that can further exhibit a function of dissolving and/or dispersing a raw material when used in combination with an ionic liquid, and is also referred to as a co-solvent for an ionic liquid.

The solvent (S) is not particularly limited, but can be appropriately selected in consideration of compatibility with the ionic liquid. When the raw material is a polysaccharide, that is, the liquid mixture (B) is a liquid mixture prepared by esterification of a polysaccharide, the solvent (S) can be appropriately selected in consideration of affinity with the polysaccharide or an esterified product of the polysaccharide, and the viscosity of a mixture of the polysaccharide and the ionic liquid. The solvent (S) is preferably a solvent that does not react with the ionic liquid, or a solvent that has high solubility in the polysaccharide and an esterified product of the polysaccharide in a state of being mixed with the ionic liquid. One type of the solvents (S) may be used alone, or two or more types thereof may be used.

Specific examples of the solvent (S) include nitriles such as acetonitrile; sulfoxides such as dimethyl sulfoxide (DMSO); sulfones such as cyclic sulfones (e.g., sulfolane); ethers such as cyclic ethers (e.g., 1,3-dioxolane, 1,4-dioxane, and tetrahydrofuran); amides such as N,N-dimethylformamide (DMF) and N,N-dimethylacetamide (DMAc); lactams such as N-methyl-2-pyrrolidone (NMP); lactones such as $\gamma$-butyrolactone; and amines such as pyridine. Among these, the solvent (S) is preferably at least one selected from the group consisting of sulfoxides, sulfones, amides, and lactams, more preferably at least one selected from the group consisting of dimethyl sulfoxide (DMSO), sulfolane, N,N-dimethylformamide (DMF), and N-methyl-2-pyrrolidone (NMP), still more preferably at least one selected from the group consisting of N,N-dimethylformamide (DMF) and N-methyl-2-pyrrolidone (NMP), and particularly preferably N-methyl-2-pyrrolidone (NMP), from the viewpoint of compatibility with the ionic liquid and the viewpoint of the solubility of an esterified product of the polysaccharide.

The solvent (S) to be used may be a solvent exemplified as a solvent (X) described below. In this case, a distillation operation can be performed without blending (adding) the solvent (X) into the liquid mixture (B1).

The above is a detailed description of the raw material, the esterifying agent, the ionic liquid, and the solvent other than the ionic liquid in the case where the liquid mixture (B) is the liquid mixture (B1).

[Distillation]

The method of the present disclosure is characterized by subjecting the liquid mixture (A) to distillation to recover an ionic liquid. Alternatively, the solvent (X) may be blended into the liquid mixture (B) to prepare the liquid mixture (A), and then the mixture is subjected to distillation. That is, at the time of distillation, the solvent (X) may be blended in the liquid mixture containing at least an ionic liquid and an organic acid. The method of blending the solvent (X) into the liquid mixture (B), i.e., the method of preparing the liquid mixture (A) is not particularly limited, and for example, the solvent (X) may be blended (added) after mixing of an ionic liquid and an organic acid, or the solvent (X) may be mixed together with an ionic liquid and an organic acid. When the liquid mixture (B) is the liquid mixture (B1), the solvent (X) may be blended in the system before the reaction, followed by various reactions, so that the solvent (X) is eventually blended in the liquid mixture (B 1). Alternatively, the solvent (A) may be prepared by blending the solvent (X) into the liquid mixture (B 1) after the

reaction.

In the method of the present disclosure, the liquid mixture (A) may be subjected to distillation as is (i.e., without pretreatment), or may be subjected to distillation after pretreatment. Such pretreatment is, for example, heat treatment of the liquid mixture (A) for a certain period of time. In a conceivable example, an organic acid (e.g., a carboxylic acid) is reacted with a solvent (e.g., an alcohol) to form a reaction product (e.g., a carboxylate ester), and then the reaction product is removed by subsequent distillation. However, in the method of the present disclosure, the liquid mixture (A) contains a specific component, and thus the organic acid and the ionic liquid can be separated from each other by distillation, and as a result the ionic liquid can be easily and efficiently recovered. That is, the method of the present disclosure exhibits the above-described effective effect without requiring the pretreatment described above. Therefore, in the method of the present disclosure, the liquid mixture (A) is preferably subjected to distillation as is (e.g., without performing a pretreatment such as a heat treatment for a certain period of time).

The operation of distillation of the liquid mixture (A) for separation of the ionic liquid is not particularly limited, and may be, for example, a continuous distillation operation in which the liquid mixture (B) and the solvent (X) are continuously charged into a distillation apparatus, or a batch distillation operation in which the solvent (X) is preliminarily blended into the liquid mixture (B) in a distillation apparatus. Alternatively, the operation may be a semi-batch distillation operation in which either the liquid mixture (B) or the solvent (X) is preliminarily charged in a distillation apparatus and the remainder is continuously charged. The distillation operation may be performed under any of reduced pressure conditions (reduced pressure type), normal pressure conditions (normal pressure type), and pressurized conditions (pressurized type), but is preferably performed under normal pressure conditions (normal pressure type) or reduced pressure conditions (reduced pressure type). In particular, the distillation operation is preferably performed not under pressurized conditions, but under reduced pressure conditions. When the distillation operation is performed not under pressurized conditions, but under reduced pressure conditions, the organic acid described below can be prevented from reacting with the ionic liquid or the solvent (X) and can be taken out as a pure organic acid. After removal of the organic acid, the ionic liquid and the solvent (X) are also prevented from reacting with each other and can be taken out as a pure ionic liquid and a pure solvent.

The temperature during distillation of the liquid mixture (A) is not particularly limited and can be appropriately determined such that the organic acid and the solvent (X) can be separated from each other. The temperature is, for example, preferably 0°C or higher, more preferably 10°C or higher, still more preferably 15°C or higher, and particularly preferably 20°C or higher. The temperature during distillation of the liquid mixture (A) is preferably 400°C or lower, more preferably 350°C or lower, still more preferably 300°C or lower, still more preferably 250°C or lower, still more preferably 200°C or lower, still more preferably 150°C or lower, still more preferably 130°C or lower, still more preferably 100°C or lower, still more preferably 95°C or lower, and still more preferably 90°C or lower. The temperature during distillation of the liquid mixture (A) is preferably within a range from 0 to 150°C, more preferably from 10 to 130°C, further preferably from 15 to 100°C, further preferably from 20 to 95°C, and particularly preferably from 20 to 90°C. The temperature during distillation of the liquid mixture (A) is also preferably from 20 to 150°C.

The pressure during distillation of the liquid mixture (A) is, for example, preferably 0.1 hPa or higher, more preferably 0.5 hPa or higher, still more preferably 0.8 hPa or higher, still more preferably 1 hPa or higher, and still more preferably 5 hPa or higher. The pressure during distillation of the liquid mixture (A) is, for example, preferably 1519 hPa or lower, more preferably 1013 hPa or lower, still more preferably 1000 hPa or lower, still more preferably 800 hPa or lower, still more preferably 500 hPa or lower, still more preferably 300 hPa or lower, still more preferably 100 hPa or lower, and still more preferably 50 hPa or lower. The pressure during distillation of the liquid mixture (A) is preferably within a range from 0.5 to 1519 hPa, more preferably from 1 to 1013 hPa, still more preferably from 5 to 100 hPa. When the pressure during distillation of the liquid mixture (A) is within the range described above, the organic acid described below does not react with the ionic liquid or the solvent (X) (for example, without causing a reaction such as an esterification reaction between an organic acid and a solvent such as an alcohol).

The time of distilling of the liquid mixture (A) is not particularly limited, and for example, a time period required to complete distillation of the organic acid out from the liquid mixture (A) can be set as the distillation time.

In the distillation operation, reflux may be performed if necessary. Particularly when the distilled-off organic acid separates from the solvent (X), the phase of the solvent (X) is preferably refluxed.

The distillation apparatus in the distillation operation is not particularly limited, and for example, a known or customary distillation apparatus can be used. The distillation apparatus to be used may be, for example, a simple distillation apparatus, a multi-stage distillation apparatus, a reduced-pressure distillation apparatus, a molecular distillation apparatus, or a steam distillation apparatus.

The ionic liquid can be separated from the liquid mixture (A) by the distillation operation described above. In general, an organic acid or a liquid mixture of an organic acid and the solvent (X) is obtained as a fraction by the distillation operation described above, and an ionic liquid or a liquid mixture containing an ionic liquid at a high concentration is obtained as a residue. From the liquid mixture containing the ionic liquid, the ionic liquid can be separated by a known or customary purification method such as further distillation, or the liquid mixture can be used as is for other applications (e.g., esterification reaction of a polysaccharide-containing biomass described below). That is, according to the method of the

present disclosure, a high-purity ionic liquid can be obtained by a simple method of distillation under easy handling conditions.

As described above, the organic acid is obtained as a fraction and the ionic liquid is obtained as a residue by the distillation operation. As a result, the ionic liquid is recovered in a form separated from the organic acid. The structural formula of the recovered ionic liquid is preferably identical to the structural formula of the ionic liquid (in the liquid mixture (A) or in the liquid mixture (B)) before distillation.

Next, an ionic liquid will be described. The ionic liquid is a liquid component separated from the organic acid by the above-described distillation operation (distillation process). When the liquid mixture (B) is the liquid mixture (B1), the ionic liquid is also a solvent for dissolving and/or dispersing a raw material (e.g., polysaccharide) (in particular, a solvent for dissolving a raw material). The ionic liquid is preferably non-volatile.

[Ionic liquid]

In the present specification, the "ionic liquid" is a salt composed of a cationic component and an anionic component, and may be any of an acidic ionic liquid, a neutral ionic liquid, and a basic ionic liquid, or may be either a water-soluble ionic liquid or a water-insoluble ionic liquid. In the present specification, the acidity or basicity of the ionic liquid can be specified by the acid dissociation constant (pKa, calculated value in vacuum) of the anion's conjugate acid, and for example, the acid dissociation constant of the basic ionic liquid is within a range from 2 to 19. One type of the above-described ionic liquids may be used alone, or two or more types thereof may be used.

• Cationic component

Examples of the cationic component in the ionic liquid include an imidazolium cation, a pyridinium cation, a pyrrolidinium cation, a piperidinium cation, a quaternary ammonium cation, and a quaternary phosphonium cation.

Examples of the imidazolium cation include a cation represented by formula (1) described below. The cation represented by formula (1) also includes a tautomer thereof and a cation represented by a structural formula in resonance with formula (1).

[Chem. 1]

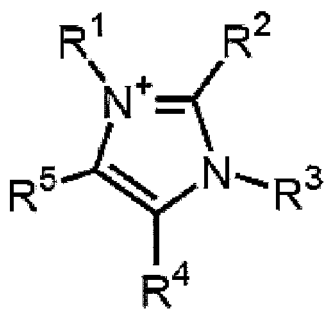

(1)

In formula (1), $R^1$ and $R^3$ are identical to or different from each other, and are each a substituted or unsubstituted alkyl group, an alkenyl group, an alkoxyalkyl group, or a substituted or unsubstituted phenyl group, and $R^2$, $R^4$, and $R^5$ are identical to or different from one another, and are each a hydrogen atom, a substituted or unsubstituted alkyl group, an alkenyl group, an alkoxyalkyl group, or a substituted or unsubstituted phenyl group.

Examples of the substituted or unsubstituted alkyl group in $R^1$ to $R^5$ include linear or branched alkyl groups having from 1 to 20 carbons (preferably from 1 to 10 carbons, more preferably from 2 to 6 carbons, still more preferably from 2 to 4 carbons), such as a methyl group, an ethyl group, an n-propyl group, an isopropyl group, a butyl group, a hexyl group, and an octyl group. A sulfo group may be bonded to an end of such an alkyl group. Examples of the alkenyl group include linear or branched alkenyl groups having from 2 to 20 carbons (preferably from 2 to 10 carbons, more preferably from 2 to 6 carbons, still more preferably from 2 to 4 carbons), such as a vinyl group, a 1-propenyl group, a 2-propenyl group, a 1-butenyl group, a 2-butenyl group, a 1-pentenyl group, a 2-pentenyl group, a 1-hexenyl group, a 2-hexenyl group, and a 1-octenyl group. Examples of the alkoxyalkyl group include linear or branched alkoxyalkyl groups having from 2 to 20 carbons (preferably from 2 to 10 carbons, more preferably from 2 to 6 carbons, still more preferably from 2 to 4 carbons), such as a methoxymethyl group, an ethoxymethyl group, a 1-methoxyethyl group, a 2-methoxyethyl group, a 1-ethoxyethyl group, and a 2-ethoxyethyl group. Further, examples of the substituted or unsubstituted phenyl group include phenyl groups that may be substituted with 1 to 2 groups selected from among a hydroxyl group, a halogen atom, a lower

alkoxy group, a lower alkenyl group, a methylsulfonyloxy group, a substituted or unsubstituted lower alkyl group, a substituted or unsubstituted amino group, a substituted or unsubstituted phenyl group, a substituted or unsubstituted phenoxy group, and a substituted or unsubstituted pyridyl group.

$R^1$ and $R^3$ are each preferably an alkyl group, an alkenyl group, or a substituted or unsubstituted phenyl group, and more preferably a linear alkyl group having from 1 to 6 carbons. Particularly preferably, one of $R^1$ and $R^3$ is a linear alkyl group having from 1 to 4 carbons, the other is a linear alkyl group having from 2 to 6 carbons, and these alkyl groups have different number of carbons. $R^2$, $R^4$, and $R^5$ are each preferably a hydrogen atom, an alkyl group, an alkenyl group, or a substituted or unsubstituted phenyl group, and more preferably a hydrogen atom or an alkyl group.

Examples of the imidazolium cation include cations of imidazolium, such as 1,3-dimethylimidazolium, 1-ethyl-3-methylimidazolium, 1-butyl-3-methylimidazolium, 1-hexyl-3-methylimidazolium, 1-octyl-3-methylimidazolium, 1-decyl-3-methylimidazolium, 1-tetradecyl-3-methylimidazolium, 1-hexadecyl-3-methylimidazolium, 1-octadecyl-3-methylimidazolium, 1-allyl-3-methylimidazolium, 1-ethyl-2,3-dimethylimidazolium, 1-butyl-2,3-dimethylimidazolium, and 1-hexyl-2,3-dimethylimidazolium. Among these, 1-ethyl-3-methylimidazolium cation ($Emim^+$) is particularly preferred.

Examples of the pyridinium cation include a cation represented by formula (2) described below. The cation represented by formula (2) also includes a tautomer thereof and a cation represented by a structural formula in resonance with formula (2).

[Chem. 2]

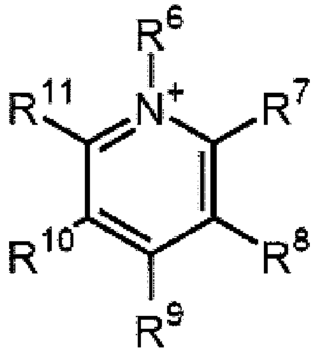

(2)

In formula (2), $R^6$ is an alkyl group, an alkenyl group, an alkoxyalkyl group, or a substituted or unsubstituted phenyl group, and $R^7$ to $R^{11}$ are identical to or different from one another and are each a hydrogen atom, an alkyl group, an alkenyl group, an alkoxyalkyl group, or a substituted or unsubstituted phenyl group.

Examples of the alkyl group, alkenyl group, alkoxyalkyl group, and substituted or unsubstituted phenyl group in $R^6$ to $R^{11}$ are the same as those described in $R^1$ to $R^5$ of the cation represented by formula (1).

$R^6$ is preferably an alkyl group, and more preferably a linear alkyl group having from 1 to 6 carbons. $R^6$ to $R^{11}$ are each preferably a hydrogen atom or an alkyl group, and more preferably a hydrogen atom.

Examples of the pyridinium cation include cations of pyridinium, such as 1-ethylpyridinium, 1-butylpyridinium, 1-hexylpyridinium, 1-butyl-4-methylpyridinium, 1-butyl-3-methylpyridinium, 1-hexyl-4-methylpyridinium, 1-hexyl-3-methylpyridinium, 1-octyl-4-methylpyridinium, 1-octyl-3-methylpyridinium, 1-butyl-3,4-dimethylpyridinium and 1-butyl-3,5-dimethylpyridinium. Among these, 1-octyl-4-methylpyridinium cation is particularly preferred.

Examples of the pyrrolidinium cation include a cation represented by formula (3) described below. The cation represented by formula (3) also includes a tautomer thereof.

[Chem. 3]

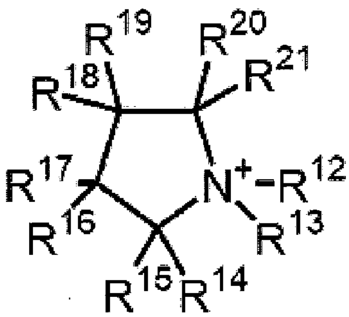

(3)

In formula (3), $R^{12}$ and $R^{13}$ are identical to or different from each other and are each an alkyl group, an alkenyl group, an alkoxyalkyl group, or a substituted or unsubstituted phenyl group, and $R^{14}$ to $R^{21}$ are identical to or different from one another and are each a hydrogen atom, an alkyl group, an alkenyl group, an alkoxyalkyl group, or a substituted or unsubstituted phenyl group.

Examples of the alkyl group, alkenyl group, alkoxyalkyl group, and substituted or unsubstituted phenyl group in $R^{12}$ to $R^{21}$ are the same as those described in $R^1$ to $R^5$ of the cation represented by formula (1).

$R^{12}$ and $R^{13}$ are each preferably an alkyl group, and more preferably a linear alkyl group having from 1 to 6 carbons. $R^{14}$ to $R^{21}$ are each preferably a hydrogen atom or an alkyl group, and more preferably a hydrogen atom.

The pyrrolidinium cation is particularly preferably 1-butyl-1-methylpyrrolidinium cation.

Examples of the piperidinium cation include a cation represented by formula (4) described below. The cation represented by formula (4) also includes a tautomer thereof.

[Chem. 4]

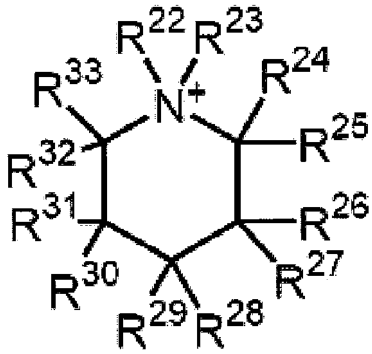

(4)

In formula (4), $R^{22}$ and $R^{23}$ are identical to or different from each other and are each an alkyl group, an alkenyl group, an alkoxyalkyl group, or a substituted or unsubstituted phenyl group, and $R^{24}$ to $R^{33}$ are identical to or different from one another and are each a hydrogen atom, an alkyl group, an alkenyl group, an alkoxyalkyl group, or a substituted or unsubstituted phenyl group.

Examples of the alkyl group, alkenyl group, alkoxyalkyl group, and substituted or unsubstituted phenyl group in $R^{22}$ to $R^{33}$ are the same as those described in $R^1$ to $R^5$ of the cation represented by formula (1).

$R^{22}$ and $R^{23}$ are each preferably an alkyl group, and more preferably a linear alkyl group having from 1 to 6 carbons. $R^{24}$ to $R^{33}$ are each preferably a hydrogen atom or an alkyl group, and more preferably a hydrogen atom.

The piperidinium cation is preferably 1-butyl-1-methylpiperidinium cation.

Examples of the quaternary ammonium cation include an ammonium cation represented by formula (5) described below. The cation represented by formula (5) also includes a tautomer thereof.

[Chem. 5]

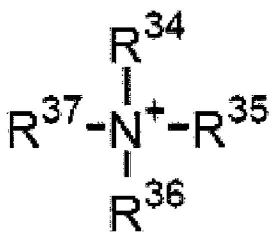

(5)

In formula (5), $R^{34}$ to $R^{37}$ are identical to or different from one another and are each an alkyl group, an alkenyl group, an alkoxyalkyl group, or a substituted or unsubstituted phenyl group.

Examples of the alkyl group in $R^{34}$ to $R^{37}$ include linear or branched alkyl groups having from 1 to 20 carbons (preferably from 1 to 10 carbons, more preferably from 2 to 6 carbons, still more preferably from 2 to 4 carbons), such as a methyl group, an ethyl group, an n-propyl group, an isopropyl group, a butyl group, a hexyl group, and an octyl group. Examples of the alkenyl group include linear or branched alkenyl groups having from 2 to 20 carbons (preferably from 2 to 10 carbons, more preferably from 2 to 6 carbons, still more preferably from 2 to 4 carbons), such as a vinyl group, a 1-propenyl group, a 2-propenyl group, a 1-butenyl group, a 2-butenyl group, a 1-pentenyl group, a 2-pentenyl group, a 1-hexenyl group, a 2-hexenyl group, and a 1-octenyl group. Examples of the alkoxyalkyl group include linear or branched alkoxyalkyl groups having from 2 to 20 carbons (preferably from 2 to 10 carbons, more preferably from 2 to 6 carbons, still more preferably from 2 to 4 carbons), such as a methoxymethyl group, an ethoxymethyl group, a 1-methoxyethyl group, a 2-methoxyethyl group, a 1-ethoxyethyl group, and a 2-ethoxyethyl group. Further, examples of the substituted or unsubstituted phenyl group include phenyl groups that may be substituted with 1 to 2 groups selected from among a hydroxyl group, a halogen atom, a lower alkoxy group, a lower alkenyl group, a methylsulfonyloxy group, a substituted or unsubstituted lower alkyl group, a substituted or unsubstituted amino group, a substituted or unsubstituted phenyl group, a substituted or unsubstituted phenoxy group, and a substituted or unsubstituted pyridyl group.

$R^{34}$ to $R^{37}$ are each preferably an alkyl group, and more preferably a linear alkyl group having from 1 to 6 carbons.

Examples of the quaternary ammonium cation include cations of ammonium, such as trimethylpropylammonium, trimethylbutylammonium, triethylmethylammonium, trioctylmethylammonium, tetramethylammonium, tetraethylammonium, tetrabutylammonium, tetrapentylammonium, and tetrahexylammonium.

Examples of the quaternary phosphonium cation include a phosphonium cation represented by formula (6) described below. The cation represented by formula (6) also includes a tautomer thereof.

[Chem. 6]

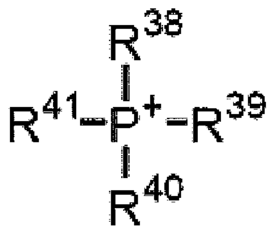

(6)

In formula (6), $R^{38}$ to $R^{41}$ are identical to or different from one another and are each an alkyl group, an alkenyl group, an alkoxyalkyl group, or a substituted or unsubstituted phenyl group.

Examples of the alkyl group in $R^{38}$ to $R^{41}$ include linear or branched alkyl groups having from 1 to 20 carbons (preferably from 1 to 10 carbons, more preferably from 2 to 6 carbons, still more preferably from 2 to 4 carbons), such as a methyl group, an ethyl group, an n-propyl group, an isopropyl group, a butyl group, a hexyl group, and an octyl group. Examples of the alkenyl group include linear or branched alkenyl groups having from 2 to 20 carbons (preferably from 2 to 10 carbons, more preferably from 2 to 6 carbons, still more preferably from 2 to 4 carbons), such as a vinyl group, a 1-propenyl group, a 2-propenyl group, a 1-butenyl group, a 2-butenyl group, a 1-pentenyl group, a 2-pentenyl group, a 1-hexenyl group, a 2-hexenyl group, and a 1-octenyl group. Examples of the alkoxyalkyl group include linear or branched alkoxyalkyl groups having from 2 to 20 carbons (preferably from 2 to 10 carbons, more preferably from 2 to 6 carbons, still more preferably from 2 to 4 carbons), such as a methoxymethyl group, an ethoxymethyl group, a 1-methoxyethyl group, a 2-methoxyethyl group, a 1-ethoxyethyl group, and a 2-ethoxyethyl group. Further, examples of the substituted or unsubstituted phenyl group include phenyl groups that may be substituted with 1 to 2 groups selected from among a hydroxyl group, a halogen atom, a lower alkoxy group, a lower alkenyl group, a methylsulfonyloxy group, a substituted or unsubstituted lower alkyl group, a substituted or unsubstituted amino group, a substituted or unsubstituted phenyl group, a substituted or unsubstituted phenoxy group, and a substituted or unsubstituted pyridyl group.

Examples of the quaternary phosphonium cation include cations of quaternary phosphonium, such as tetramethylphosphonium, tetraethylphosphonium, tetrabutylphosphonium, tetrahexylphosphonium, tetraoctylphosphonium, triethylmethylphosphonium, and tributylethylphosphonium.

• Anionic component

Examples of the anionic component in the ionic liquid include a halogen anion, a pseudohalogen anion, a carboxylate anion, a phosphate-based anion, an amino acid anion, a phenolate, a pyrimidine olate, a tetrafluoroborate ion ($BF^{4-}$), a sulfomethyl ion ($CH_3SO_3^-$), methyl phosphonate, a sulfate ion, and $PF_6^-$.

The halogen anion is not particularly limited, and examples thereof include a fluoride ion ($F^-$), a chloride ion ($Cl^-$), an iodine ion ($I^-$), and a bromide ion ($Br^-$).

The pseudohalogen anion is not particularly limited, and examples thereof include a cyan anion, a thiocyanate anion, a cyanate anion, a fulminate anion, and an azide anion.

The carboxylate anion is not particularly limited, and examples thereof include a monocarboxylate anion or dicarboxylate anion having from 1 to 18 carbons. Examples of the carboxylate anion include a formate anion, an acetate anion, a propionate anion, a butyrate anion, a valerate anion, a fumarate anion, an oxalate anion, a lactate anion, and a pyruvate anion. The anionic component of the ionic liquid preferably contains a carboxylate anion ($OAc^-$) from the

viewpoint that the ionic liquid can be easily and efficiently recovered.

The phosphate-based anion is not particularly limited, and examples thereof include a phosphate anion and a phosphate ester anion having from 1 to 40 carbons. Examples of the phosphate ester anion include methyl phosphate monoester anion, octyl phosphate monoester anion, octyl phosphate diester anion, lauryl phosphate monoester anion, lauryl phosphate diester anion, stearyl phosphate monoester anion, stearyl phosphate diester anion, eicosyl phosphate monoester anion, and eicosyl phosphate diester anion.

Each of the ionic liquids described above may be a commercially available product. Alternatively, it can also be produced by a known technique.

The ionic liquid is any combination of the cationic component and the anionic component. Preferred is, for example, an ionic liquid containing an imidazolium cation or a quaternary ammonium cation as a cationic component. The imidazolium cation is particularly preferred, and the reason for this is probably due to the skeleton of formula (1) in the relationship between affinity and separation efficiency in the distillation operation. Also preferred is an ionic liquid containing a carboxylate anion as an anionic component. More specifically, the ionic liquid is more preferably at least one selected from the group consisting of 1-ethyl-3-methylimidazolium acetate (EmimOAc), 1-ethyl-2,3-dimethylimidazolium acetate (EDmimOAc), 1-butyl-3-methylimidazolium acetate, 1-hexyl-3-methylimidazolium acetate, and tetrabutylammonium acetate. The ionic liquid is further preferably at least one selected from the group consisting of 1-ethyl-3-methylimidazolium acetate (EmimOAc) and 1-ethyl-2,3-dimethylimidazolium acetate (EDmimOAc), and is particularly preferably 1-ethyl-3-methylimidazolium acetate (EmimOAc).

The content of the ionic liquid in the liquid mixture (A) is preferably 0.1 wt.% or more, more preferably 0.3 wt.% or more, still more preferably 0.5 wt.% or more, particularly preferably 1 wt.% or more, and most preferably 2 wt.% or more.

When the solvent (X) is at least one selected from the group consisting of a linear or branched monoalcohol having an HSP value [$(MPa)^{0.5}$] of from 18.5 to 21.5 at 25°C and a monoalcohol having an alicyclic skeleton and having an HSP value [$(MPa)^{0.5}$] of from 21.6 to 23 at 25°C, the content of the ionic liquid in the liquid mixture (A) is preferably 9.9 wt.% or less, more preferably 9.5 wt.% or less, still more preferably 9 wt.% or less, still more preferably 8 wt.% or less, still more preferably 7 wt.% or less, and still more preferably 5 wt.% or less. In particular, when the solvent (X) is at least one selected from the group consisting of cyclohexanol, 1-hexanol, 1-heptanol, 2-octanol, and 2-ethylhexanol, the content of the ionic liquid in the liquid mixture is more preferably within a range from 0.3 to 9 wt.%, still more preferably within a range from 0.5 to 8 wt.%, and particularly preferably within a range from 1 to 7 wt.%.

When the solvent (X) is water, the content of the ionic liquid in the liquid mixture (A) is preferably 45 wt.% or less, more preferably 40 wt.% or less, still more preferably 30 wt.% or less, still more preferably 20 wt.% or less, still more preferably 9.9 wt.% or less, still more preferably 9.5 wt.% or less, still more preferably 9 wt.% or less, and still more preferably 5 wt.% or less. When the amount of the ionic liquid blended in the liquid mixture (A) is within the range described above, the ionic liquid can be easily and efficiently recovered from the liquid mixture (A).

[Organic acid]

Next, an organic acid will be described. The organic acid is a component to be separated from the ionic liquid by the above-described distillation operation.

Examples of the organic acid include carboxylic acids such as formic acid, acetic acid, propionic acid, and trifluoroacetic acid (preferably $C_{1-18}$ carboxylic acids, more preferably $C_{1-10}$ carboxylic acids, still more preferably $C_{1-6}$ carboxylic acids, particularly preferably $C_{2-4}$ carboxylic acids). The carboxylic acid is also preferably a $C_{9-18}$ carboxylic acid, and is also preferably a $C_{1-7}$ carboxylic acid. One type of the above-described organic acids may be used alone, or two or more types thereof may be used.

The organic acid may be derived from the esterifying agent. In this case, the esterifying agent is, for example, an acid anhydride. For example, when an ionic liquid is used as a solvent and an acid anhydride is used as an esterifying agent for esterification of a polysaccharide, the resulting liquid mixture (B2) may contain the ionic liquid and the acid anhydride and/or an organic acid derived from the acid anhydride. The method of the present disclosure can be suitably used for recovering the ionic liquid from the liquid mixture (B2) as described above.

When the organic acid is a carboxylic acid, the content of a $C_{1-6}$ carboxylic acid relative to the carboxylic acid (total amount) is, for example, preferably 70 wt.% or more, more preferably 80 wt.% or more, still more preferably 90 wt.% or more, still more preferably 95 wt.% or more, and particularly preferably 99 wt.% or more. In this case, the upper limit of the content of the $C_{1-6}$ carboxylic acid relative to the carboxylic acid (total amount) is preferably, for example, 100 wt.%.

The molar ratio of the ionic liquid to the organic acid (ionic liquid:organic acid) contained in the liquid mixture (A) is not particularly limited, and is, for example, preferably within a range from 1:100 to 100: 1, more preferably from 10:90 to 90:10, still more preferably from 20:80 to 80:20, and particularly preferably from 30:70 to 70:30. When the ratio of the ionic liquid to the organic acid contained in the liquid mixture (A) is within the range described above, the ionic liquid tends to be easily and efficiently recovered from the liquid mixture (A). When the solvent (X) is blended into the liquid mixture (B) to prepare the liquid mixture (A), the molar ratio of the ionic liquid to the organic acid contained in the liquid mixture (B) is also

preferably in the range described above.

The content of the organic acid in the liquid mixture (A) is, for example, preferably 0.01 wt.% or more, more preferably 0.1 wt.% or more, still more preferably 0.3 wt.% or more, and particularly preferably 0.4 wt.% or more. When the solvent (X) is at least one selected from the group consisting of a linear or branched monoalcohol having an HSP value [$(MPa)^{0.5}$] of from 18.5 to 21.5 at 25°C and a monoalcohol having an alicyclic skeleton and having an HSP value [$(MPa)^{0.5}$] of from 21.6 to 23 at 25°C, the content of the organic acid in the liquid mixture (A) is, for example, preferably 4.9 wt.% or less, more preferably 4.5 wt.% or less, still more preferably 4 wt.% or less, even more preferably 3 wt.% or less, and particularly preferably 2 wt.% or less. In particular, when the solvent (X) is at least one selected from the group consisting of cyclohexanol, 1-hexanol, 1-heptanol, 2-octanol, and 2-ethylhexanol, the content of the organic acid in the liquid mixture (A) is more preferably within a range from 0.1 to 4 wt.%, still more preferably within a range from 0.3 to 3 wt.%, and particularly preferably within a range from 0.4 to 2 wt.%.

When the solvent (X) is water, the content of the organic acid in the liquid mixture (A) is, for example, preferably 30 wt.% or less, more preferably 20 wt.% or less, still more preferably 15 wt.% or less, still more preferably 10 wt.% or less, still more preferably 7 wt.% or less, still more preferably 4.9 wt.% or less, and still more preferably 4 wt.% or less. The content of the organic acid in the liquid mixture (A) is preferably 0.01 wt.% or more, more preferably 0.05 wt.% or more, still more preferably 0.1 wt.% or more, and still more preferably 0.2 wt.% or more. When the amount of the organic acid in the liquid mixture (A) is within the range described above, the ionic liquid and the organic acid can be separated from the liquid mixture (A), and the ionic liquid can be easily and efficiently recovered.

[Solvent (X)]

Next, a solvent (X) will be described. The solvent (X) may be already contained in the liquid mixture (B), may be newly added to the liquid mixture (B), or may be already contained in the liquid mixture (B) and may be further added to the liquid mixture (B). The solvent (X) has a function of separating an ionic liquid and an organic acid when the liquid mixture (A) is subjected to a distillation operation. The solvent (X) is not particularly limited as long as the above-described organic acid is excluded. Examples of the solvent (X) include polar solvents, such as water, alcohols, alkanediols, amides, lactams, sulfoxides, sulfones, ethers, ketones, nitriles, and esters. One type of the solvents (X) may be used alone, or two or more types thereof may be used.

Examples of the alcohols include linear or branched monoalcohols such as methanol, ethanol, 1-propanol, 2-propanol, 1-butanol, 2-butanol, isobutanol, tert-butanol, tert-pentanol, 1-hexanol, 2-hexanol, 2-ethylhexanol, tert-butylcarbinol, 1-heptanol, 2-heptanol, 1-octanol, 2-octanol, and allyl alcohol (preferably $C_{1-12}$ monoalcohols, more preferably $C_{3-10}$ monoalcohols, particularly preferably $C_{5-8}$ monoalcohols); and monoalcohols having an alicyclic skeleton, such as cyclohexanol (preferably $C_{3-12}$ monoalcohols, particularly preferably $C_{5-10}$ monoalcohols). Examples of the alkanediols include 1,2-$C_{1-12}$ alkanediols such as 1,2-butanediol, 1,2-pentanediol, 1,2-hexanediol, and 1,2-heptanediol; and 1,3-$C_{1-12}$ alkanediols such as 1,3-hexanediol, 1,3-heptanediol, and 2-ethyl-1,3-hexanediol. Examples of the amides include N,N-dimethylformamide (DMF) and N,N-dimethylacetamide (DMAc). Examples of the lactams include 2-pyrrolidone, N-methyl-2-pyrrolidone (NMP), and N-vinyl-2-pyrrolidone. Examples of the sulfoxides include dimethyl sulfoxide (DMSO). Examples of the sulfones include cyclic sulfones such as sulfolane. Examples of the ethers include diethyl ether, 1,3-dioxolane, 1,4-dioxane, and tetrahydrofuran. Examples of the ketones include acetone, methyl ethyl ketone, and methyl isobutyl ketone. Examples of the nitriles include acetonitrile. Examples of the esters include methyl acetate, ethyl acetate, and butyl acetate.

The HSP value [$(MPa)^{0.5}$] of the solvent (X) at 25°C is not particularly limited, and is, for example, preferably 15 or more, more preferably 18 or more, and still more preferably 20 or more. The upper limit of the HSP value is not particularly limited, but is, for example, preferably 80, more preferably 60, still more preferably 50, still more preferably 40, still more preferably 30, still more preferably 25, still more preferably 22, and still more preferably 21. When the HSP value of the solvent (X) at 25°C is within the range described above, the ionic liquid tends to be easily and efficiently recovered from the liquid mixture (A). The HSP value [$(MPa)^{0.5}$] described above is based on the Hansen method (Allan F. M. Barton. Chem. Rev., 1975, 75 (6), pp 731-753) and is expressed by the following equation.

$$\text{HSP value} = (\delta_D^2 + \delta_P^2 + \delta_H^2)^{0.5}$$

Dispersion term $\delta_D$: the energy from dispersion forces between molecules
Polarity term $\delta_P$: the energy from dipolar intermolecular forces between molecules
Hydrogen bond term $\delta_H$: the energy from hydrogen bonds between molecules.

The HSP value may be obtained by using various estimation methods (e.g., atomic group contribution method and computer simulation), and can be calculated by using, for example, computer software Hansen Solubility Parameters

in Practice (HSPiP).

The solvent (X) is preferably water, an alcohol, an amide, a lactam, or a sulfoxide from the viewpoint of easily and efficiently recovering the ionic liquid from the liquid mixture (A). More preferred examples of the solvent (X) include a linear or branched monoalcohol having an HSP value [$(MPa)^{0.5}$] ranging from 15 to 30 (still more preferably from 18 to 25, even more preferably from 18.5 to 21.5, particularly preferably from 19 to 21) at 25°C; a monoalcohol having an alicyclic skeleton and having an HSP value [$(MPa)^{0.5}$] ranging from 15 to 30 (still more preferably from 18 to 25, even more preferably from 20 to 23, particularly preferably from 21.6 to 23) at 25°C; an amide having an HSP value [$(MPa)^{0.5}$] ranging from 15 to 30 (still more preferably from 18 to 28, even more preferably from 20 to 27, particularly preferably from 23 to 26) at 25°C; a lactam having an HSP value [$(MPa)^{0.5}$] ranging from 15 to 30 (still more preferably from 18 to 28, even more preferably from 20 to 27, particularly preferably from 22.5 to 25) at 25°C; and a sulfoxide having an HSP value [$(MPa)^{0.5}$] ranging from 15 to 30 (still more preferably from 20 to 29, even more preferably from 23 to 28) at 25°C.

In the method of the present disclosure, the solvent (X) is preferably at least one (referred to as a solvent (X1)) selected from the group consisting of linear or branched monoalcohols having an HSP value [$(MPa)^{0.5}$] ranging from 18.5 to 21.5 at 25°C and monoalcohols having an alicyclic skeleton and having an HSP value [$(MPa)^{0.5}$] ranging from 21.6 to 23 at 25°C. Among these, at least one (referred to as a solvent (X2)) selected from the group consisting of cyclohexanol, 1-hexanol, 1-heptanol, 2-octanol, and 2-ethylhexanol is more preferred, and at least one (referred to as a solvent (X3)) selected from the group consisting of 1-hexanol, 1-heptanol, 2-octanol, and 2-ethylhexanol is still more preferred. The solvent (X1) has a very strong ability to separate the ionic liquid from the organic acid, and thus the ionic liquid can be easily and effectively recovered.

As the solvent (X), the above-described solvent (X1), (X2), or (X3) (hereinafter collectively referred to as a solvent (XN)) is preferably used in combination with the following solvent (X'1), (X'2), (X'3), (X'4), or (X'5) (hereinafter collectively referred to as a solvent (X'N)). From the viewpoint of the compatibility with an ionic liquid and from the viewpoint of the solubility of an esterified product of a polysaccharide, the solvent (X) to be used in combination with the solvent (XN) is preferably at least one (referred to as a solvent (X'1)) selected from the group consisting of amides, lactams, sulfoxides, and sulfones, more preferably at least one (referred to as a solvent (X'2)) selected from the group consisting of amides having an HSP value [$(MPa)^{0.5}$] ranging from 23 to 26 at 25°C, lactams having an HSP value [$(MPa)^{0.5}$] ranging from 22.5 to 25 at 25°C, sulfoxides having an HSP value [$(MPa)^{0.5}$] ranging from 23 to 28 at 25°C, and sulfones having an HSP value [$(MPa)^{0.5}$] ranging from 24 to 28 at 25°C, further preferably at least one (referred to as solvent (X'3)) selected from the group consisting of N,N-dimethylformamide (DMF), N-methyl-2-pyrrolidone (NMP), and dimethyl sulfoxide (DMSO), further preferably at least one (referred to as solvent (X'4)) selected from the group consisting of N,N-dimethylformamide (DMF) and N-methyl-2-pyrrolidone (NMP), and particularly preferably N-methyl-2-pyrrolidone (NMP) (solvent (X'5)). That is, the solvent (X) to be used in combination with the solvent (XN) is preferably the solvent (X'N).

The solvent (X'N) may be already contained in the liquid mixture (B), may be newly added to the liquid mixture (B), or may be already contained in the liquid mixture (B) and may be further added to the liquid mixture (B). That is, when the liquid mixture (A) contains the solvent (X'N), the process in which the solvent (X'N) is blended may be any of the above-described processes. In a case where the solvent (X'N) is already contained in the liquid mixture (B) from the beginning, the solvent (X'N) may be contained as the solvent (S). That is, in a case where the liquid mixture (B) is the liquid mixture (B1), the solvent (S) itself serves as the solvent (X'N) upon preparation of the liquid mixture (A).

The boiling point of the solvent (X) is not particularly limited, and is, for example, preferably within a range from 20 to 300°C, more preferably from 40 to 280°C, and still more preferably from 50 to 250°C. When the solvent (X) contains two or more solvents, the boiling point described above may be that of a mixture of the two or more solvents.

The latent heat of vaporization of the solvent (X) is not particularly limited, and is, for example, preferably within a range from 40 to 1000 kcal/kg, more preferably from 60 to 800 kcal/kg, and still more preferably from 80 to 600 kcal/kg. When the solvent (X) contains two or more solvents, the latent heat of vaporization may be that of a mixture of the two or more solvents.

The content of the solvent (X) contained in the liquid mixture (A) is not particularly limited, and is, for example, preferably 9.09 wt.% or more, more preferably 16.7 wt.% or more, still more preferably 23.1 wt.% or more, even more preferably 28.6 wt.% or more, and particularly preferably 33.3 wt.% or more. The content of the solvent (X) contained in the liquid mixture (A) is not particularly limited, and is, for example, preferably 99.1 wt.% or less, more preferably 98.8 wt.% or less, still more preferably 98.4 wt.% or less, still more preferably 98.0 wt.% or less, still more preferably 97.6 wt.% or less, still more preferably 90 wt.% or less, and still more preferably 80 wt.% or less. When the solvent (X) is blended in the liquid mixture (B), the content of the solvent (X) contained in the resulting liquid mixture (A) is preferably adjusted to satisfy the range described above.

The amount of the solvent (X) blended in the liquid mixture (B) is, for example, preferably within a range from 10 to 10000 parts by weight, more preferably from 20 to 8000 parts by weight, still more preferably from 30 to 6000 parts by weight, still more preferably from 40 to 5000 parts by weight, and still more preferably from 50 to 4000 parts by weight, per 100 parts by weight of the liquid mixture (B). When the amount of the solvent (X) blended in the liquid mixture (B) is within the range described above, the ionic liquid tends to be easily and efficiently recovered from the liquid mixture (A).

The amount of the solvent (X) blended in the liquid mixture (B) per 1 mol of the ionic liquid is, for example, preferably 1 mol or more, more preferably 3 mol or more, still more preferably 5 mol or more, still more preferably 7 mol or more, still more preferably 10 mol or more, and still more preferably 15 mol or more. The amount of the solvent (X) blended in the liquid mixture (B) per 1 mol of the ionic liquid is, for example, preferably 500 mol or less, more preferably 300 mol or less, still more preferably 200 mol or less, still more preferably 100 mol or less, and still more preferably 80 mol or less. The preferred range of the amount of the solvent (X) blended in the liquid mixture (B) per 1 mol of the ionic liquid is from 1 to 500 mol, more preferably from 3 to 300 mol, still more preferably from 5 to 200 mol, even more preferably from 7 to 100 mol, and particularly preferably from 10 to 80 mol. When the amount of the solvent (X) is within the range described above, the ionic liquid in the liquid mixture (A) tends to be recovered at high yield.
The preferred amounts of the solvent (XN) and the solvent (X'N) will be described for a case where the solvent (X) contained in the liquid mixture (A) is a combination of the solvent (XN) and the solvent (X'N). The amount of the solvent (XN) per 1 mol of the solvent (X'N) is preferably 1 mol or more, more preferably 5 mol or more, still more preferably 10 mol or more. The amount of the solvent (XN) per 1 mol of the solvent (X'N) is preferably 500 mol or less, more preferably 300 mol or less, still more preferably 200 mol or less. The amount of the solvent (XN) per 1 mol of the solvent (X'N) is preferably within a range from 1 to 500 mol, more preferably from 5 to 300 mol, still more preferably from 10 to 200 mol. The preferred ranges of the amounts of the solvent (XN) and the solvent (X'N) are the same as those described above also in a case where the solvents are blended in the liquid mixture (B).

[Composition and composition for distillation]

The present embodiment includes a composition containing an ionic liquid, an organic acid, and a solvent (X). When this composition is subjected to distillation under normal pressure or reduced pressure, the ionic liquid and the organic acid are separated from each other, and a liquid mixture containing the ionic liquid and the solvent (X) is suitably produced. The composition in the present embodiment is preferably for distillation. Hereinafter, the composition for distillation will be described.
A preferred embodiment of the ionic liquid contained in the composition for distillation is the same as the preferred embodiment described above in the section [Ionic liquid]. A preferred embodiment of the organic acid contained in the composition for distillation is the same as the preferred embodiment described above in the section [Organic acid]. A preferred embodiment of the solvent (X) contained in the composition for distillation is the same as the preferred embodiment described above in the section [Solvent (X)]. Preferred distillation conditions of the composition for distillation are the same as those in the preferred embodiment described above in the section [Distillation]. In particular, the above-described liquid mixture (A) can be regarded as the composition for distillation, and the types and amounts of preferred components, distillation conditions, and the like can be referred to the liquid mixture (A). The composition for distillation is prepared by blending the solvent (X) into the liquid mixture (B) containing the ionic liquid and the organic acid. Therefore, the types and amounts of preferred components, distillation conditions, and the like of the composition for distillation can be referred not only to the liquid mixture (A), but also to the liquid mixture (B).
Configurations, a combination of the configurations, and the like in each of the embodiments described above are an example, and various additions, omissions, substitutions, and other changes of the configurations may be made as appropriate without departing from the spirit of the present disclosure. The present disclosure is not limited by the embodiments and is limited only by the claims.

Examples

The present disclosure will next be described in more detail by way of Examples, but the present disclosure is not limited to the Examples.
The specifications of the evaporator used in Examples are as follows.

Product name: Rotary Evaporator N-1110
Manufacturer: TOKYO RIKAKIKAI CO., LTD.

The specifications of the NMR apparatus used in Examples are as follows.

Product name: JNM-ECZ600R
Manufacturer: JEOL Ltd.

[Example 1: water/acetic acid/EmimOAc]

A mixed solution containing 3.92 g of water (HSP value = 47.8 $(MPa)^{0.5}$), 0.26 g of acetic acid, and 0.74 g of

EmimOAc (water:acetic acid:EmimOAc (molar proportions) = 50:1:1) was provided. The mixed solution was subjected to evaporation at 25 hPa and 60°C for 30 minutes. The residue in a flask was sampled, and the composition of the residue in the flask was calculated from NMR quantification results and found to be as follows: acetic acid:EmimOAc (molar ratio) = 0.58:1. The results indicated that blending of water enabled 41.4 wt.% of acetic acid to be removed by distillation.

[Example 2: methanol/acetic acid/EmimOAc]

A mixed solution containing 6.96 g of methanol (HSP value = 29.4 $(MPa)^{0.5}$), 0.28 g of acetic acid, and 0.74 g of EmimOAc (methanol:acetic acid:EmimOAc (molar proportions) = 50:1:1) was provided. The mixed solution was subjected to evaporation at 25 hPa and 60°C for 3 hours. The residue in a flask was sampled, and the composition of the residue in the flask was calculated from NMR quantification results and found to be as follows: acetic acid:EmimOAc (molar ratio) = 0.96:1. The results indicated that blending of methanol enabled 10.5 wt.% of acetic acid to be removed by distillation.

[Example 3: DMSO/acetic acid/EmimOAc]

A mixed solution containing 17.00 g of DMSO (dimethyl sulfoxide, HSP value = 26.7 $(MPa)^{0.5}$), 0.26 g of acetic acid, and 0.75 g of EmimOAc (DMSO:acetic acid:EmimOAc (molar proportions) = 50:1:1) was provided. The mixed solution was subjected to evaporation at 10 hPa and 95°C for 16 hours. The residue in a flask was sampled, and the composition of the residue in the flask was calculated from NMR quantification results and found to be as follows: acetic acid:EmimOAc (molar ratio) = 0.64:1. The results indicated that blending of DMSO enabled 36.8 wt.% of acetic acid to be removed by distillation.

[Example 4: ethanol/acetic acid/EmimOAc]

A mixed solution containing 10.00 g of ethanol (HSP value = 26.5 $(MPa)^{0.5}$), 0.26 g of acetic acid, and 0.75 g of EmimOAc (ethanol:acetic acid:EmimOAc (molar proportions) = 50:1:1) was provided. The mixed solution was subjected to evaporation at 10 hPa and 60°C for 1 hour. The residue in a flask was sampled, and the composition of the residue in the flask was calculated from NMR quantification results and found to be as follows: acetic acid:EmimOAc (molar ratio) = 0.91:1. The results indicated that blending of ethanol enabled 7.6 wt.% of acetic acid to be removed by distillation.

[Example 5: DMF/acetic acid/EmimOAc]

A mixed solution containing 15.85 g of DMF (N,N-dimethylformamide, HSP value = 24.9 $(MPa)^{0.5}$), 0.26 g of acetic acid, and 0.74 g of EmimOAc (DMF:acetic acid:EmimOAc (molar proportions) = 50:1:1) was provided. The mixed solution was subjected to evaporation at 10 hPa and 65°C for 16 hours. The residue in a flask was sampled, and the composition of the residue in the flask was calculated from NMR quantification results and found to be as follows: acetic acid:EmimOAc (molar ratio) = 0.76:1. The results indicated that blending of DMF enabled 23.2 wt.% of acetic acid to be removed by distillation.

[Example 6: 1-propanol/acetic acid/EmimOAc]

A mixed solution containing 13.07 g of 1-propanol (HSP value = 24.6 $(MPa)^{0.5}$), 0.27 g of acetic acid, and 0.74 g of EmimOAc (1-propanol:acetic acid:EmimOAc (molar proportions) = 50:1:1) was provided. The mixed solution was subjected to evaporation at 10 hPa and 60°C for 3 hours. The residue in a flask was sampled, and the composition of the residue in the flask was calculated from NMR quantification results and found to be as follows: acetic acid:EmimOAc (molar ratio) = 0.88:1. The results indicated that blending of 1-propanol enabled 17.6 wt.% of acetic acid to be removed by distillation.

[Example 7: 2-propanol/acetic acid/EmimOAc]

A mixed solution containing 13.05 g of 2-propanol (HSP value = 23.6 $(MPa)^{0.5}$), 0.27 g of acetic acid, and 0.73 g of EmimOAc (2-propanol:acetic acid:EmimOAc (molar proportions) = 50:1:1) was prepared. The mixed solution was subjected to evaporation at 10 hPa and 60°C for 30 minutes. The residue in a flask was sampled, and the composition of the residue in the flask was calculated from NMR quantification results and found to be as follows: acetic acid:EmimOAc (molar ratio) = 0.95:1. The results indicated that blending of 2-propanol enabled 8.6 wt.% of acetic acid to be removed by distillation.

[Example 8: 1-butanol/acetic acid/EmimOAc]

A mixed solution containing 15.83 g of 1-butanol (HSP value = 23.2 $(MPa)^{0.5}$), 0.27 g of acetic acid, and 0.74 g of EmimOAc (1-butanol:acetic acid:EmimOAc (molar proportions) = 50:1:1) was provided. The mixed solution was subjected to evaporation at 10 hPa and 60°C for 2.5 hours. The residue in a flask was sampled, and the composition of the residue in the flask was calculated from NMR quantification results and found to be as follows: acetic acid:EmimOAc (molar ratio) = 0.80:1. The results indicated that blending of 1-butanol enabled 27.8 wt.% of acetic acid to be removed by distillation.

[Example 9: NMP/acetic acid/EmimOAc]

A mixed solution containing 21.56 g of NMP (N-methyl-2-pyrrolidone, HSP value = 23.0 $(MPa)^{0.5}$), 0.27 g of acetic acid, and 0.77 g of EmimOAc (NMP:acetic acid:EmimOAc (molar proportions) = 50:1:1) was provided. The mixed solution was subjected to evaporation at 10 hPa and 105°C for 16 hours. The residue in a flask was sampled, and the composition of the residue in the flask was calculated from NMR quantification results and found to be as follows: acetic acid:EmimOAc (molar ratio) = 0.47:1. The results indicated that blending of NMP enabled 54.8 wt.% of acetic acid to be removed by distillation.

[Example 10: cyclohexanol/acetic acid/EmimOAc]

A mixed solution containing 21.77 g of cyclohexanol (HSP value = 22.4 $(MPa)^{0.5}$), 0.26 g of acetic acid, and 0.75 g of EmimOAc (cyclohexanol:acetic acid:EmimOAc (molar proportions) = 50:1:1) was provided. The mixed solution was subjected to evaporation at 10 hPa and 90°C for 16 hours. The residue in a flask was sampled, and the composition of the residue in the flask was calculated from NMR quantification results and found to be as follows: acetic acid:EmimOAc (molar ratio) = 0.22:1. The results indicated that blending of cyclohexanol enabled 77.7 wt.% of acetic acid to be removed by distillation.

[Example 11: 2-butanol/acetic acid/EmimOAc]

A mixed solution containing 16.12 g of 2-butanol (HSP value = 22.2 $(MPa)^{0.5}$), 0.27 g of acetic acid, and 0.74 g of EmimOAc (2-butanol:acetic acid:EmimOAc (molar proportions) = 50:1:1) was provided. The mixed solution was subjected to evaporation at 10 hPa and 60°C for 2.5 hours. The residue in a flask was sampled, and the composition of the residue in the flask was calculated from NMR quantification results and found to be as follows: acetic acid:EmimOAc (molar ratio) = 0.93:1. The results indicated that blending of 2-butanol enabled 8.5 wt.% of acetic acid to be removed by distillation.

[Example 12: tert-butyl alcohol/acetic acid/EmimOAc]

A mixed solution containing 16.1 g of tert-butyl alcohol (HSP value = 21.8 $(MPa)^{0.5}$), 0.26 g of acetic acid, and 0.75 g of EmimOAc (tert-butyl alcohol:acetic acid:EmimOAc (molar proportions) = 50:1:1) was provided. The mixed solution was subjected to evaporation at 10 hPa and 60°C for 1 hour. The residue in a flask was sampled, and the composition of the residue in the flask was calculated from NMR quantification results and found to be as follows: acetic acid:EmimOAc (molar ratio) = 0.94:1. The results indicated that blending of tert-butyl alcohol enabled 4.9 wt.% of acetic acid to be removed by distillation.

[Example 13: 1-hexanol/acetic acid/EmimOAc]

A mixed solution containing 22.20 g of 1-hexanol (HSP value = 21.0 $(MPa)^{0.5}$), 0.27 g of acetic acid, and 0.73 g of EmimOAc (1-hexanol:acetic acid:EmimOAc (molar proportions) = 50:1:1) was provided. The mixed solution was subjected to evaporation at 10 hPa and 85°C for 2.5 hours. The residue in a flask was sampled, and the composition of the residue in the flask was calculated from NMR quantification results and found to be as follows: acetic acid:EmimOAc (molar ratio) = 0.18:1. The results indicated that blending of 1-hexanol enabled 82.8 wt.% of acetic acid to be removed by distillation.

[Example 14: 1-heptanol/acetic acid/EmimOAc]

A mixed solution containing 25.44 g of 1-heptanol (HSP value = 20.5 $(MPa)^{0.5}$), 0.27 g of acetic acid, and 0.73 g of EmimOAc (1-heptanol:acetic acid:EmimOAc (molar proportions) = 50:1:1) was provided. The mixed solution was

subjected to evaporation at 10 hPa and 90°C for 2.5 hours. The residue in a flask was sampled, and the composition of the residue in the flask was calculated from NMR quantification results and found to be as follows: acetic acid:EmimOAc (molar ratio) = 0.004:1. The results indicated that blending of 1-heptanol enabled 99.6 wt.% of acetic acid to be removed by distillation.

[Example 15: 2-octanol/acetic acid/EmimOAc]

A mixed solution containing 28.25 g of 2-octanol (HSP value = 20.1 $(MPa)^{0.5}$), 0.26 g of acetic acid, and 0.75 g of EmimOAc (2-octanol:acetic acid:EmimOAc (molar proportions) = 50:1:1) was provided. The mixed solution was subjected to evaporation at 10 hPa and 100°C for 16 hours. The residue in a flask was sampled, and the composition of the residue in the flask was calculated from NMR quantification results and found to be as follows: acetic acid:EmimOAc (molar ratio) = 0.002:1. The results indicated that blending of 2-octanol enabled 99.8 wt.% of acetic acid to be removed by distillation.

[Example 16: 2-ethylhexanol/acetic acid/EmimOAc]

A mixed solution containing 28.29 g of 2-ethylhexanol (HSP value = 20.1 $(MPa)^{0.5}$), 0.26 g of acetic acid, and 0.75 g of EmimOAc (2-ethylhexanol:acetic acid:EmimOAc (molar proportions) = 50:1:1) was provided. The mixed solution was subjected to evaporation at 10 hPa and 105°C for 16 hours. The residue in a flask was sampled, and the composition of the residue in the flask was calculated from NMR quantification results and found to be as follows: acetic acid:EmimOAc (molar ratio) = 0.01:1. The results indicated that blending of 2-ethylhexanol enabled 98.9 wt.% of acetic acid to be removed by distillation.

[Example 17: water/acetic acid/EmimOAc]

A mixed solution containing 0.82 g of water (HSP value = 47.8 $(MPa)^{0.5}$), 0.26 g of acetic acid, and 0.74 g of EmimOAc (water:acetic acid:EmimOAc (molar proportions) = 10:1:1) was provided. The mixed solution was subjected to evaporation with an evaporator at 25 hPa and 60°C for 30 minutes. The residue in a flask was sampled, and the composition of the residue in the flask was calculated from NMR quantification results and found to be as follows: acetic acid:EmimOAc (molar ratio) = 0.94:1. The results indicated that blending of water enabled 5.3 wt.% of acetic acid to be removed by distillation.

[Example 18: water/acetic acid/EmimOAc]

A mixed solution containing 1.62 g of water (HSP value = 47.8 $(MPa)^{0.5}$), 0.26 g of acetic acid, and 0.74 g of EmimOAc (water:acetic acid:EmimOAc (molar proportions) = 20:1:1) was provided. The mixed solution was subjected to evaporation with an evaporator at 25 hPa and 60°C for 30 minutes. The residue in a flask was sampled, and the composition of the residue in the flask was calculated from NMR quantification results and found to be as follows: acetic acid:EmimOAc (molar ratio) = 0.74:1. The results indicated that blending of water enabled 26.4 wt.% of acetic acid to be removed by distillation.

[Example 19: water/acetic acid/EmimOAc]

A mixed solution containing 7.97 g of water (HSP value = 47.8 $(MPa)^{0.5}$), 0.26 g of acetic acid, and 0.74 g of EmimOAc (water:acetic acid:EmimOAc (molar proportions) = 100:1:1) was provided. The mixed solution was subjected to evaporation with an evaporator at 25 hPa and 60°C for 30 minutes. The residue in a flask was sampled, and the composition of the residue in the flask was calculated from NMR quantification results and found to be as follows: acetic acid:EmimOAc (molar ratio) = 0.41:1. The results indicated that blending of water enabled 59.1 wt.% of acetic acid to be removed by distillation.

[Example 20; water/acetic acid/EmimOAc]

A mixed solution containing 15.66 g of water (HSP value = 47.8 $(MPa)^{0.5}$), 0.26 g of acetic acid, and 0.74 g of EmimOAc (water:acetic acid:EmimOAc (molar proportions) = 200:1:1) was provided. The mixed solution was subjected to evaporation with an evaporator at 25 hPa and 60°C for 30 minutes. The residue in a flask was sampled, and the composition of the residue in the flask was calculated from NMR quantification results and found to be as follows: acetic acid:EmimOAc (molar ratio) = 0.32:1. The results indicated that blending of water enabled 68.1 wt.% of acetic acid to be removed by distillation.

[Example 21: water/acetic acid/EmimOAc]

A mixed solution containing 23.49 g of water (HSP value = 47.8 $(MPa)^{0.5}$), 0.26 g of acetic acid, and 0.74 g of EmimOAc (water:acetic acid:EmimOAc (molar proportions) = 300:1:1) was provided. The mixed solution was subjected to evaporation with an evaporator at 25 hPa and 60°C for 30 minutes. The residue in a flask was sampled, and the composition of the residue in the flask was calculated from NMR quantification results and found to be as follows: acetic acid:EmimOAc (molar ratio) = 0.27:1. The results indicated that blending of water enabled 73.3 wt.% of acetic acid to be removed by distillation.

[Example 22: 2-octanol/NMP/acetic acid/EmimOAc]

There were provided 38.1 parts by weight of 2-octanol, 1 part by weight of NMP, 0.17 parts by weight of acetic acid, and 1 part by weight of EmimOAc. Next, these four components were mixed to prepare a mixed solution (2-octanol:NMP:acetic acid:EmimOAc (molar proportions) = 50:1.72:0.50:1). Subsequently, the mixed solution was subjected to a batch distillation. The batch distillation was performed by using a 20-tray Oldershaw column. The batch distillation was performed by the following process: (1) the pressure was reduced from normal pressure in a stepwise manner until the column top pressure reached 26.6 hPa; (2) after the pressure was stabilized, the temperature was increased in a total reflux state; (3) after the temperature in the column was stabilized, the total reflux state was retained for 1 hour; (4) the reflux ratio was changed to 5, and the distillation was initiated; and (5) a sample was taken 30 minutes after initiation of the distillation, and the composition of the sample was analyzed. The oil bath temperature was set to 150°C. As a result, acetic acid was completely removed by distillation 30 minutes after initiation of the distillation following the total reflux operation. That is, blending of 2-octanol and NMP enabled the entire amount of acetic acid to be removed.

[Example 23]

The structural formula of recovered EmimOAc in Example 1 is the same as the structural formula of EmimOAc before distillation (i.e., before evaporation).

[Example 24]

When the EmimOAc (1 g) recovered in each of Examples 1, 13, 14, 15, 16, and 22 is mixed with cellulose (0.18 g, trade name "Avicel PH-101" available from Sigma-Aldrich, number average polymerization degree: 105), and the mixture is stirred, the cellulose is dissolved in the EmimOAc. This phenomenon demonstrates that the ionic liquid recovered by the method of the present disclosure can be reused.

[Comparative Example 1: without use of solvent]

A liquid mixture containing 52 g of acetic acid and 148 g of EmimOAc (acetic acid:EmimOAc (molar ratio) = 1.0:1.0) was prepared and heated to 145°C at 53 hPa. However, no distillate was obtained under the above-mentioned conditions, which indicated that a gas-liquid equilibrium state was not achieved. That is, it was found that acetic acid cannot be removed by distillation without blending a solvent into the liquid mixture.

[Comparative Example 2: without use of solvent]

A liquid mixture containing 69 g of acetic acid and 131 g of EmimOAc (acetic acid:EmimOAc (molar ratio) = 1.5:1.0) was prepared and heated to 145°C at 53 hPa. However, no distillate was obtained under the above-mentioned conditions, which indicated that a gas-liquid equilibrium state was not achieved. That is, it was found that acetic acid cannot be removed by distillation without blending a solvent into the liquid mixture.

[Comparative Example 3: without use of solvent]

A liquid mixture containing 80 g of acetic acid and 120 g of EmimOAc (acetic acid:EmimOAc (molar ratio) = 1.9:1.0) was prepared and heated to 145°C at 53 hPa. However, no distillate was obtained under the above-mentioned conditions, which indicated that a gas-liquid equilibrium state was not achieved. That is, it was found that acetic acid cannot be removed by distillation without blending a solvent into the liquid mixture.

[Supplementary description of Examples 1 to 22]

The weight percent of EmimOAc and the weight percent of acetic acid in the liquid mixture before the evaporating operation (evaporation) under reduced pressure in each of Examples 1 to 21 are described below. In addition, the weight percent of EmimOAc and the weight percent of acetic acid in the liquid mixture before the batch distillation in Example 22 are described below.

- Example 1: EmimOAc: 15.0 wt.%, acetic acid: 5.31 wt.%
- Example 2: EmimOAc: 9.29 wt.%, acetic acid: 3.28 wt.%
- Example 3: EmimOAc: 4.11 wt.%, acetic acid: 1.45 wt.%
- Example 4: EmimOAc: 6.72 wt.%, acetic acid: 2.37 wt.%
- Example 5: EmimOAc: 4.38 wt.%, acetic acid: 1.55 wt.%
- Example 6: EmimOAc: 5.26 wt.%, acetic acid: 1.86 wt.%
- Example 7: EmimOAc: 5.26 wt.%, acetic acid: 1.86 wt.%
- Example 8: EmimOAc: 4.32 wt.%, acetic acid: 1.53 wt.%
- Example 9: EmimOAc: 3.28 wt.%, acetic acid: 1.16 wt.%
- Example 10: EmimOAc: 3.25 wt.%, acetic acid: 1.15 wt.%
- Example 11: EmimOAc: 4.32 wt.%, acetic acid: 1.53 wt.%
- Example 12: EmimOAc: 4.32 wt.%, acetic acid: 1.53 wt.%
- Example 13: EmimOAc: 3.19 wt.%, acetic acid: 1.12 wt.%
- Example 14: EmimOAc: 2.82 wt.%, acetic acid: 0.99 wt.%
- Example 15: EmimOAc: 2.52 wt.%, acetic acid: 0.89 wt.%
- Example 16: EmimOAc: 2.52 wt.%, acetic acid: 0.89 wt.%
- Example 17: EmimOAc: 41.5 wt.%, acetic acid: 14.6 wt.%
- Example 18: EmimOAc: 28.8 wt.%, acetic acid: 10.2 wt.%
- Example 19: EmimOAc: 8.38 wt.%, acetic acid: 2.96 wt.%
- Example 20: EmimOAc: 4.44 wt.%, acetic acid: 1.57 wt.%
- Example 21: EmimOAc: 3.02 wt.%, acetic acid: 1.07 wt.%
- Example 22: EmimOAc: 2.48 wt.%, acetic acid was 0.42 wt.%

To summarize the above, configurations of the present invention and variations thereof will be described below.

[1] A method for recovering an ionic liquid, the method being characterized by including subjecting a liquid mixture (A) containing an ionic liquid, an organic acid, and a solvent (provided that an organic acid is excluded) (i.e., "solvent (X)") to distillation.

[2] The method for recovering an ionic liquid according to [1], characterized in that a solvent (provided that an organic acid is excluded) is blended into a liquid mixture (B) containing an ionic liquid and an organic acid, and the liquid mixture is subjected to distillation.

[3] The method for recovering an ionic liquid according to [2], wherein the liquid mixture (B) is a liquid mixture containing a reaction product obtained upon esterification of a specific raw material with an esterifying agent by using an ionic liquid as a solvent.

[4] The method for recovering an ionic liquid according to [3], wherein the raw material is a polysaccharide (e.g., at least one selected from the group consisting of cellulose, hemicellulose, xylan, mannan, glucomannan, glucuronoxylan, starch, amylose, amylopectin, glycogen, dextrin, pectin, chitin, chitosan, agarose, carrageenan, isolichenan, laminaran, lichenan, glucan, inulin, levan, fructan, galactan, arabinan, pentosan, alginic acid, pectinic acid, protuberonic acid, colominic acid, porphyran, fucoidan, ascophyllan, locust bean gum, guar gum, tamarind gum, tara gum, and gum arabic).

[5] The method for recovering an ionic liquid according to [3] or [4], wherein the esterifying agent is one or more selected from the group consisting of a chain ester compound, a cyclic ester compound, an unsaturated aldehyde, a saturated aldehyde, an acid halide, an acid anhydride, and allyl alcohol.

[6] The method for recovering an ionic liquid according to [5], wherein the acid anhydride is one or more selected from the group consisting of acetic anhydride, propionic anhydride, butyric anhydride, valeric anhydride, caproic anhydride, enanthic anhydride, caprylic anhydride, pelargonic anhydride, capric anhydride, lauric anhydride, myristic anhydride, palmitic anhydride, stearic anhydride, oleic anhydride, linoleic anhydride, linolenic anhydride, benzoic anhydride, phthalic anhydride, maleic anhydride, and succinic anhydride.

[7] The method for recovering an ionic liquid according to any one of [2] to [6], wherein the liquid mixture (B) is a liquid mixture containing a solvent other than an ionic liquid (solvent (S)).

[8] The method for recovering an ionic liquid according to [7], wherein the solvent (S) is at least one selected from the

group consisting of nitriles such as acetonitrile; sulfoxides such as dimethyl sulfoxide (DMSO); sulfones such as cyclic sulfones (e.g., sulfolane); ethers such as cyclic ethers (e.g., 1,3-dioxolane, 1,4-dioxane, and tetrahydrofuran); amides such as N,N-dimethylformamide (DMF) and N,N-dimethylacetamide (DMAc); lactams such as N-methyl-2-pyrrolidone (NMP); lactones such as γ-butyrolactone; and amines such as pyridine; at least one selected from the group consisting of sulfoxides, sulfones, amides, and lactams; at least one selected from the group consisting of dimethyl sulfoxide (DMSO), sulfolane, N,N-dimethylformamide (DMF), and N-methyl-2-pyrrolidone (NMP); at least one selected from the group consisting of N,N-dimethylformamide (DMF) and N-methyl-2-pyrrolidone (NMP); or N-methyl-2-pyrrolidone (NMP).

[9] The method for recovering an ionic liquid according to any one of [1] to [8], wherein the liquid mixture (A) is subjected to a distillation operation without a pretreatment.

[10] The method for recovering an ionic liquid according to any one of [1] to [9], wherein the temperature during distillation of the liquid mixture (A) is 0°C or higher, 10°C or higher, 15°C or higher, 20°C or higher, 400°C or lower, 350°C or lower, 300°C or lower, 250°C or lower, 200°C or lower, 150°C or lower, 130°C or lower, 100°C or lower, 95°C or lower, 90°C or lower, from 0 to 150°C, from 10 to 130°C, from 15 to 100°C, from 20 to 95°C, from 20 to 90°C, and/or from 20 to 150°C.

[11] The method for recovering an ionic liquid according to any one of [1] to [10], wherein the pressure during distillation of the liquid mixture (A) is 0.1 hPa or higher, 0.5 hPa or higher, 0.8 hPa or higher, 1 hPa or higher, 5 hPa or higher, 1519 hPa or lower, 1013 hPa or lower, 1000 hPa or lower, 800 hPa or lower, 500 hPa or lower, 300 hPa or lower, 100 hPa or lower, 50 hPa or lower, from 0.5 to 1519 hPa, from 1 to 1013 hPa, and/or from 5 to 100 hPa.

[12] The method for recovering an ionic liquid according to any one of [1] to [11], wherein the structural formula of a recovered ionic liquid is the same as the structural formula of the ionic liquid (in the liquid mixture (A) or the liquid mixture (B)) before distillation.

[13] The method for recovering an ionic liquid according to any one of [1] to [12], wherein the ionic liquid contains, as a cationic component, at least one selected from the group consisting of an imidazolium cation, a pyridinium cation, a pyrrolidinium cation, a piperidinium cation, a quaternary ammonium cation, and a quaternary phosphonium cation.

[14] The method for recovering an ionic liquid according to [13], wherein the imidazolium cation is a cation represented by formula (1) (wherein $R^1$ and $R^3$ are identical to or different from each other and are each a substituted or unsubstituted alkyl group, an alkenyl group, an alkoxyalkyl group, or a substituted or unsubstituted phenyl group; and $R^2$, $R^4$, and $R^5$ are identical to or different from one another and are each a hydrogen atom, a substituted or unsubstituted alkyl group, an alkenyl group, an alkoxyalkyl group, or a substituted or unsubstituted phenyl group).

[15] The method for recovering an ionic liquid according to [13] or [14], wherein the pyridinium cation is a cation represented by formula (2) (wherein $R^6$ is an alkyl group, an alkenyl group, an alkoxyalkyl group, or a substituted or unsubstituted phenyl group; and $R^7$ to $R^{11}$ are identical to or different from one another and are each a hydrogen atom, an alkyl group, an alkenyl group, an alkoxyalkyl group, or a substituted or unsubstituted phenyl group).

[16] The method for recovering an ionic liquid according to any one of [13] to [15], wherein the pyrrolidinium cation is a cation represented by formula (3) (wherein $R^{12}$ and $R^{13}$ are identical to or different from each other and are each an alkyl group, an alkenyl group, an alkoxyalkyl group, or a substituted or unsubstituted phenyl group; and $R^{14}$ to $R^{21}$ are identical to or different from one another and are each a hydrogen atom, an alkyl group, an alkenyl group, an alkoxyalkyl group, or a substituted or unsubstituted phenyl group).

[17] The method for recovering an ionic liquid according to any one of [13] to [16], wherein the piperidinium cation is a cation represented by formula (4) (wherein $R^{22}$ and $R^{23}$ are identical to or different from each other and are each an alkyl group, an alkenyl group, an alkoxyalkyl group, or a substituted or unsubstituted phenyl group; and $R^{24}$ to $R^{33}$ are identical to or different from one another and are each a hydrogen atom, an alkyl group, an alkenyl group, an alkoxyalkyl group, or a substituted or unsubstituted phenyl group).

[18] The method for recovering an ionic liquid according to any one of [13] to [17], wherein the quaternary ammonium cation is an ammonium cation represented by formula (5) (wherein $R^{34}$ to $R^{37}$ are identical to or different from one another and are each an alkyl group, an alkenyl group, an alkoxyalkyl group, or a substituted or unsubstituted phenyl group).

[19] The method for recovering an ionic liquid according to any one of [13] to [18], wherein the quaternary phosphonium cation is a phosphonium cation represented by formula (6) (wherein $R^{38}$ to $R^{41}$ are identical to or different from one another and are each an alkyl group, an alkenyl group, an alkoxyalkyl group, or a substituted or unsubstituted phenyl group).

[20] The method for recovering an ionic liquid according to any one of [1] to [19], wherein the ionic liquid contains, as an anionic component, at least one selected from the group consisting of a halogen anion, a pseudohalogen anion, a carboxylate anion, a phosphate-based anion, an amino acid anion, a phenolate, a pyrimidine olate, a tetrafluoroborate ion ($BF^{4-}$), a sulfomethyl ion ($CH_3SO_3^-$), methyl phosphonate, a sulfate ion, and $PF_6^-$.

[21] The method for recovering an ionic liquid according to any one of [1] to [20], wherein the ionic liquid contains a carboxylate anion as an anionic component.

[22] The method for recovering an ionic liquid according to any one of [1] to [21], wherein the ionic liquid contains an imidazolium cation or a quaternary ammonium cation as a cationic component.

[23] The method for recovering an ionic liquid according to any one of [1] to [22], wherein the content of the ionic liquid in the liquid mixture (A) is 0.1 wt.% or more, 0.3 wt.% or more, 0.5 wt.% or more, 1 wt.% or more, or 2 wt.% or more.

[24] The method for recovering an ionic liquid according to any one of [1] to [23], wherein when the solvent (X) is at least one selected from the group consisting of a linear or branched monoalcohol having an HSP value [$(MPa)^{0.5}$] of from 18.5 to 21.5 at 25°C and a monoalcohol having an alicyclic skeleton and having an HSP value [$(MPa)^{0.5}$] of from 21.6 to 23 at 25°C, the content of the ionic liquid in the liquid mixture (A) is 9.9 wt.% or less, 9.5 wt.% or less, 9 wt.% or less, 8 wt.% or less, 7 wt.% or less, or 5 wt.% or less.

[25] The method for recovering an ionic liquid according to any one of [1] to [24], wherein when the solvent (X) is at least one selected from the group consisting of cyclohexanol, 1-hexanol, 1-heptanol, 2-octanol, and 2-ethylhexanol, the content of the ionic liquid in the liquid mixture is within a range from 0.3 to 9 wt.%, from 0.5 to 8 wt.%, and/or from 1 to 7 wt.%.

[26] The method for recovering an ionic liquid according to any one of [1] to [25], wherein when the solvent (X) is water, the content of the ionic liquid in the liquid mixture (A) is 45 wt.% or less, 40 wt.% or less, 30 wt.% or less, 20 wt.% or less, 9.9 wt.% or less, 9.5 wt.% or less, 9 wt.% or less, or 5 wt.% or less.

[27] The method for recovering an ionic liquid according to any one of [1] to [26], wherein the organic acid is a carboxylic acid, a $C_{1-18}$ carboxylic acid, a $C_{1-10}$ carboxylic acid, a $C_{1-6}$ carboxylic acid, a $C_{2-4}$ carboxylic acid, a $C_{9-18}$ carboxylic acid, and/or a $C_{1-7}$ carboxylic acid.

[28] The method for recovering an ionic liquid according to any one of [1] to [27], wherein the organic acid is at least one selected from the group consisting of formic acid, acetic acid, propionic acid, and trifluoroacetic acid.

[29] The method for recovering an ionic liquid according to any one of [1] to [28], wherein the organic acid is a carboxylic acid, and the content of the $C_{1-6}$ carboxylic acid relative to the carboxylic acid (total amount) is 70 wt.% or more, 80 wt.% or more, 90 wt.% or more, 95 wt.% or more, or 99 wt.% or more.

[30] The method for recovering an ionic liquid according to any one of [1] to [29], wherein the molar ratio of the ionic liquid and the organic acid (ionic liquid:organic acid) contained in the liquid mixture (A) or the liquid mixture (B) is within a range from 1:100 to 100: 1, from 10:90 to 90: 10, from 20:80 to 80:20, or from 30:70 to 70:30.

[31] The method for recovering an ionic liquid according to any one of [1] to [30], wherein the content of the organic acid in the liquid mixture (A) is 0.01 wt.% or more, 0.1 wt.% or more, 0.3 wt.% or more, or 0.4 wt.% or more.

[32] The method for recovering an ionic liquid according to any one of [1] to [31], wherein when the solvent (X) is at least one selected from the group consisting of a linear or branched monoalcohol having an HSP value [$(MPa)^{0.5}$] of from 18.5 to 21.5 at 25°C and a monoalcohol having an alicyclic skeleton and having an HSP value [$(MPa)^{0.5}$] of from 21.6 to 23 at 25°C, the content of the organic acid in the liquid mixture (A) is 4.9 wt.% or less, 4.5 wt.% or less, 4 wt.% or less, 3 wt.% or less, or 2 wt.% or less.

[33] The method for recovering an ionic liquid according to any one of [1] to [32], wherein when the solvent (X) is at least one selected from the group consisting of cyclohexanol, 1-hexanol, 1-heptanol, 2-octanol, and 2-ethylhexanol, the content of the organic acid in the liquid mixture (A) is within a range from 0.1 to 4 wt.%, from 0.3 to 3 wt.%, or from 0.4 to 2 wt.%.

[34] The method for recovering an ionic liquid according to any one of [1] to [33], wherein when the solvent (X) is water, the content of the organic acid in the liquid mixture (A) is 30 wt.% or less, 20 wt.% or less, 15 wt.% or less, 10 wt.% or less, 7 wt.% or less, 4.9 wt.% or less, 4 wt.% or less, 0.01 wt.% or more, 0.05 wt.% or more, 0.1 wt.% or more, and/or 0.2 wt.% or more.

[35] The method for recovering an ionic liquid according to any one of [1] to [34], wherein the solvent (X) is at least one selected from the group consisting of water, an alcohol, an alkanediol, an amide, a lactam, a sulfoxide, a sulfone, an ether, a ketone, a nitrile, and an ester.

[36] The method for recovering an ionic liquid according to [35], wherein the alcohol is a linear or branched monoalcohol such as methanol, ethanol, 1-propanol, 2-propanol, 1-butanol, 2-butanol, isobutanol, tert-butanol, tert-pentanol, 1-hexanol, 2-hexanol, 2-ethylhexanol, tert-butylcarbinol, 1-heptanol, 2-heptanol, 1-octanol, 2-octanol, or allyl alcohol, a $C_{1-12}$ monoalcohol, a $C_{3-10}$ monoalcohol, or a $C_{5-8}$ monoalcohol.

[37] The method for recovering an ionic liquid according to [35] or [36], wherein the alcohol is a monoalcohol having an alicyclic skeleton such as cyclohexanol, a $C_{3-12}$ monoalcohol, or a $C_{5-10}$ monoalcohol.

[38] The method for recovering an ionic liquid according to any one of [35] to [37], wherein the alkanediol is a 1,2-$C_{1-12}$ alkanediol such as 1,2-butanediol, 1,2-pentanediol, 1,2-hexanediol, or 1,2-heptanediol.

[39] The method for recovering an ionic liquid according to any one of [35] to [38], wherein the alkanediol is a 1,3-$C_{1-12}$ alkanediol such as 1,3-hexanediol, 1,3-heptanediol, or 2-ethyl-1,3-hexanediol.

[40] The method for recovering an ionic liquid according to any one of [35] to [39], wherein the amide is at least one selected from the group consisting of N,N-dimethylformamide (DMF) and N,N-dimethylacetamide (DMAc).

[41] The method for recovering an ionic liquid according to any one of [35] to [40], wherein the lactam is at least one

selected from the group consisting of 2-pyrrolidone, N-methyl-2-pyrrolidone (NMP), and N-vinyl-2-pyrrolidone.

[42] The method for recovering an ionic liquid according to any one of [35] to [41], wherein the sulfoxide is dimethyl sulfoxide (DMSO).

[43] The method for recovering an ionic liquid according to any one of [35] to [42], wherein the sulfone is a cyclic sulfone such as sulfolane.

[44] The method for recovering an ionic liquid according to any one of [35] to [43], wherein the ether is at least one selected from the group consisting of diethyl ether, 1,3-dioxolane, 1,4-dioxane, and tetrahydrofuran.

[45] The method for recovering an ionic liquid according to any one of [35] to [44], wherein the ketone is at least one selected from the group consisting of acetone, methyl ethyl ketone, and methyl isobutyl ketone.

[46] The method for recovering an ionic liquid according to any one of [35] to [45], wherein the nitrile is acetonitrile.

[47] The method for recovering an ionic liquid according to any one of [35] to [46], wherein the ester is at least one selected from the group consisting of methyl acetate, ethyl acetate, and butyl acetate.

[48] The method for recovering an ionic liquid according to any one of [1] to [47], wherein the HSP value [$(MPa)^{0.5}$] of the solvent (X) at 25°C is 15 or more, 18 or more, 20 or more, 80 or less, 60 or less, 50 or less, 40 or less, 30 or less, 25 or less, 22 or less, and/or 21 or less.

[49] The method for recovering an ionic liquid according to any one of [1] to [48], wherein the solvent (X) is at least one selected from the group consisting of water, an alcohol, an amide, a lactam, and a sulfoxide.

[50] The method for recovering an ionic liquid according to any one of [1] to [49], wherein the solvent (X) is a linear or branched monoalcohol having an HSP value [$(MPa)^{0.5}$] at 25°C of from 15 to 30, from 18 to 25, from 18.5 to 21.5, or from 19 to 21.

[51] The method for recovering an ionic liquid according to any one of [1] to [50], wherein the solvent (X) is a monoalcohol having an alicyclic skeleton and having an HSP value [$(MPa)^{0.5}$] at 25°C of from 15 to 30, from 18 to 25, from 20 to 23, or from 21.6 to 23.

[52] The method for recovering an ionic liquid according to any one of [1] to [51], wherein the solvent (X) is an amide having an HSP value [$(MPa)^{0.5}$] at 25°C of from 15 to 30, from 18 to 28, from 20 to 27, or from 23 to 26.

[53] The method for recovering an ionic liquid according to any one of [1] to [52], wherein the solvent (X) is a lactam having an HSP value [$(MPa)^{0.5}$] at 25°C of from 15 to 30, from 18 to 28, from 20 to 27, or from 22.5 to 25.

[54] The method for recovering an ionic liquid according to any one of [1] to [53], wherein the solvent (X) is a sulfoxide having an HSP value [$(MPa)^{0.5}$] at 25°C of from 15 to 30, from 20 to 29, or from 23 to 28.

[55] The method for recovering an ionic liquid according to any one of [1] to [54], wherein the solvent (X) is at least one (solvent (X1)) selected from the group consisting of a linear or branched monoalcohol having an HSP value [$(MPa)^{0.5}$] of from 18.5 to 21.5 at 25°C and a monoalcohol having an alicyclic skeleton and having an HSP value [$(MPa)^{0.5}$] of from 21.6 to 23 at 25°C; at least one (solvent (X2)) selected from the group consisting of cyclohexanol, 1-hexanol, 1-heptanol, 2-octanol, and 2-ethylhexanol; or at least one (solvent (X3)) selected from the group consisting of 1-hexanol, 1-heptanol, 2-octanol, and 2-ethylhexanol.

[56] The method for recovering an ionic liquid according to any one of [1] to [55], wherein the solvent (X) is at least one (solvent (X'1)) selected from the group consisting of an amide, a lactam, a sulfoxide, and a sulfone; at least one (solvent (X'2)) selected from the group consisting of an amide having an HSP value [$(MPa)^{0.5}$] of from 23 to 26 at 25°C, a lactam having an HSP value [$(MPa)^{0.5}$] of from 22.5 to 25 at 25°C, a sulfoxide having an HSP value [$(MPa)^{0.5}$] of from 23 to 28 at 25°C, and a sulfone having an HSP value [$(MPa)^{0.5}$] of from 24 to 28 at 25°C; at least one (solvent (X'3)) selected from the group consisting of N,N-dimethylformamide (DMF), N-methyl-2-pyrrolidone (NMP), and dimethyl sulfoxide (DMSO); at least one (solvent (X'4)) selected from the group consisting of N,N-dimethylformamide (DMF) and N-methyl-2-pyrrolidone (NMP); or N-methyl-2-pyrrolidone (NMP) (solvent (X'5)).

[57] The method for recovering an ionic liquid according to [56], wherein, as the solvent (X), the solvent (X1), (X2), or (X3) (collectively referred to as a solvent (XN)) is used in combination with the solvent (X'1), (X'2), (X'3), (X'4), or (X'5) (collectively referred to as a solvent (X'N)).

[58] The method for recovering an ionic liquid according to any one of [1] to [57], wherein the content of the solvent (X) contained in the liquid mixture (A) is 9.09 wt.% or more, 16.7 wt.% or more, 23.1 wt.% or more, 28.6 wt.% or more, 33.3 wt.% or more, 99.1 wt.% or less, 98.8 wt.% or less, 98.4 wt.% or less, 98.0 wt.% or less, 97.6 wt.% or less, 90 wt.% or less, and/or 80 wt.% or less.

[59] The method for recovering an ionic liquid according to any one of [1] to [58], wherein the amount of the solvent (X) blended into the liquid mixture (B) is from 10 to 10000 parts by weight, from 20 to 8000 parts by weight, from 30 to 6000 parts by weight, from 40 to 5000 parts by weight, or from 50 to 4000 parts by weight relative to 100 parts by weight of the liquid mixture (B).

[60] The method for recovering an ionic liquid according to any one of [1] to [59], wherein the amount of the solvent (X) blended into the liquid mixture (B) per 1 mol of the ionic liquid is 1 mol or more, 3 mol or more, 5 mol or more, 7 mol or more, 10 mol or more, 15 mol or more, 500 mol or less, 300 mol or less, 200 mol or less, 100 mol or less, 80 mol or less, from 1 to 500 mol, from 3 to 300 mol, from 5 to 200 mol, from 7 to 100 mol, and/or from 10 to 80 mol.

[61] The method for recovering an ionic liquid according to any one of [1] to [60], wherein when the solvent (X) contained in the liquid mixture (A) is a combination of the solvent (XN) and the solvent (X'N), the amount of the solvent (XN) per 1 mol of the solvent (X'N) is 1 mol or more, 5 mol or more, 10 mol or more, 500 mol or less, 300 mol or less, 200 mol or less, from 1 to 500 mol, from 5 to 300 mol is, or from 10 to 200 mol.
[62] The method for recovering an ionic liquid according to any one of [1] to [61], wherein the solvent (X) is at least one selected from the group consisting of cyclohexanol, 1-hexanol, 1-heptanol, 2-octanol, and 2-ethylhexanol, and the content of the ionic liquid in the liquid mixture (A) is within a range from 0.3 to 9 wt.%.
[63] The method for recovering an ionic liquid according to any one of [1] to [62], wherein the pressure during the distillation is 1519 hPa or less.
[64] A composition for distillation, the composition containing an ionic liquid, an organic acid, and a solvent (provided that an organic acid is excluded), wherein

the solvent in the composition for distillation is at least one selected from the group consisting of cyclohexanol, 1-hexanol, 1-heptanol, 2-octanol, and 2-ethylhexanol;
the ionic liquid is at least one selected from the group consisting of EmimOAc and EDmimOAc;
the organic acid is acetic acid;
the content of the ionic liquid in the composition is within a range from 0.3 to 9 wt.%; and
the content of the organic acid in the composition is within a range from 0.1 to 4 wt.%.

Industrial Applicability

According to the method for recovering an ionic liquid of the present disclosure, an ionic liquid can be easily and efficiently recovered from a liquid mixture containing the ionic liquid and an organic acid.

## Claims

1. A method for recovering an ionic liquid, the method comprising subjecting a liquid mixture (A) containing an ionic liquid, an organic acid, and a solvent (provided that an organic acid is excluded) to distillation.

2. The method for recovering an ionic liquid according to claim 1, wherein the solvent (provided that an organic acid is excluded) is blended into a liquid mixture (B) containing the ionic liquid and the organic acid, and the liquid mixture is subjected to distillation.

3. The method for recovering an ionic liquid according to claim 1 or 2, wherein the solvent is a polar solvent.

4. The method for recovering an ionic liquid according to any one of claims 1 to 3, wherein the solvent has an HSP value [$(MPa)^{0.5}$] of 15 or more at 25°C.

5. The method for recovering an ionic liquid according to any one of claims 1 to 4, wherein the ionic liquid contains an imidazolium cation or a quaternary ammonium cation as a cationic component.

6. The method for recovering an ionic liquid according to any one of claims 1 to 5, wherein the ionic liquid contains a carboxylate anion as an anionic component.

7. The method for recovering an ionic liquid according to any one of claims 1 to 6, wherein the organic acid is a carboxylic acid.

8. The method for recovering an ionic liquid according to any one of claims 1 to 7, wherein the solvent is at least one selected from the group consisting of cyclohexanol, 1-hexanol, 1-heptanol, 2-octanol, and 2-ethylhexanol, and a content of the ionic liquid in the liquid mixture (A) is from 0.3 to 9 wt.%.

9. The method for recovering an ionic liquid according to any one of claims 1 to 8, wherein a pressure during the distillation is 1519 hPa or less.

# INTERNATIONAL SEARCH REPORT

International application No.
**PCT/JP2023/010486**

**A. CLASSIFICATION OF SUBJECT MATTER**

***C07D 233/58***(2006.01)i; ***C08B 3/06***(2006.01)i; ***C08B 3/22***(2006.01)i
FI: C07D233/58; C08B3/06; C08B3/22

According to International Patent Classification (IPC) or to both national classification and IPC

**B. FIELDS SEARCHED**

Minimum documentation searched (classification system followed by classification symbols)

C07D233/58; C08B3/06; C08B3/22

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2011-530643 A (EASTMAN CHEMIAL COMPANY) 22 December 2011 (2011-12-22) fig. 2, paragraphs [0147]-[0153], [0160], example 29 | 1-7, 9 |
| A | | 8 |
| X | JOGUNOLA, O et al. Carbohydrate Polymers. 2016, vol. 135, pp. 341-348 abstract, p. 347, left column, line 23 to right column, line 2 | 1-7, 9 |
| A | | 8 |
| X | SHI, J. et al. J. Chem. Eng. Data. 2013, vol. 58, pp. 197-202 abstract, p. 198, right column, bottom 16 lines, p. 200, right column, lines 13-36, table 4 | 1-7, 9 |
| A | | 8 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 May 2023** | **30 May 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/010486**

| Patent document cited in search report | Publication date (day/month/year) | Patent family member(s) | Publication date (day/month/year) |
|---|---|---|---|
| JP 2011-530643 A | 22 December 2011 | US 2010/0029927 A1<br>fig. 2, paragraphs [0176]-[0183], [0090], example 29<br>US 2008/0194808 A1<br>US 2012/0101269 A1<br>US 2017/0204201 A1<br>US 2011/0213138 A1<br>US 2012/0123112 A1<br>US 2013/0190485 A1<br>US 2016/0108137 A1<br>US 2012/0095207 A1<br>US 2012/0142910 A1<br>US 2008/0194807 A1<br>US 2008/0194834 A1<br>WO 2010/019244 A1<br>WO 2010/019245 A1<br>WO 2008/100566 A1<br>WO 2008/100569 A1<br>WO 2008/100577 A1<br>EP 3239179 A1<br>KR 10-2011-0043757 A<br>CN 102124031 A<br>CN 102977216 A<br>CN 103923352 A<br>KR 10-2009-0109106 A<br>KR 10-2009-0109107 A<br>KR 10-2009-0109556 A<br>CN 101657428 A<br>CN 101657470 A<br>CN 101668779 A<br>CN 102603643 A<br>CN 102675207 A<br>CN 103467606 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016068053 A
- JP 2015096255 A
- JP 2012144441 A
- JP 2010184902 A
- JP 2008523005 A


### Non-patent literature cited in the description


- **ALLAN F. M. BARTON**. *Chem. Rev.*, 1975, vol. 75 (6), 731-753